# EUROPEAN PATENT APPLICATION

(11) **EP 2 832 745 A1**
(43) Date of publication of application: **04.02.2015**
(21) Application number: 13769311.5
(22) Date of filing: 29.03.2013
(51) Int. Cl.: C07K 7/56, C12P 21/04, A61K 38/12, A61P 31/10

(54) **HIGH PURITY CYCLOPEPTIDE COMPOUND AS WELL AS PREPARATION METHOD AND USE THEREOF**

(30) Priority: 30.03.2012 CN 201210090338
(71) Applicant: Shanghai Techwell Biopharmaceutical Co., Ltd, Shanghai 201108 (CN)
(72) Inventor: LIU, Shidong, Shanghai 201108 (CN); ZHANG, Zhaoli, Shanghai 201108 (CN); WANG, Xiusheng, Shanghai 201108 (CN); ZHANG, Xiao, Shanghai 201108 (CN); JIAO, Guangjun, Shanghai 201108 (CN); HE, Bingming, Shanghai 201108 (CN); TANG, Zhijun, Shanghai 201108 (CN); JI, Xiaoming, Shanghai 201108 (CN)
(74) Representative: Germinario, Claudio
(86) International application number: PCT/CN2013/073508
(87) International publication number: WO 2013/143497

(57) **Abstract**

Disclosed is a high purity cyclopeptide compound, the chemical structure of which is represented by formula (I). R represents H or a cation capable of forming a pharmaceutically acceptable salt, the purity being greater than or equal to 99.0%. Further disclosed are preparation method and use of the high purity cyclopeptide compound.

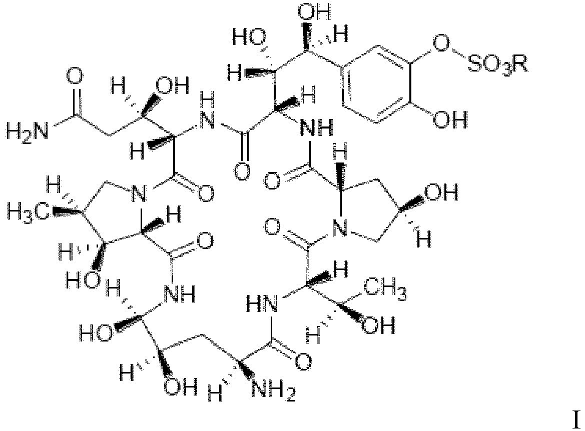

## Description

### Technicla field

The present invention relates to a high purity cyclopeptide compound and the preparation method, and also relates to the use of such high purity cyclopeptide compound.

### Background

Fungal infection has become the leading cause for high morbidity and mortality in immunodeficient patients. During the past 20 years, the incidence of fungal infection increased significantly. People at high-risk of fungal infections includes critical patients, surgical patients and those patients suffering from HIV infection, leukemia and other tumors. Patients with organ transplant are also at high risk of fungal infection.

Echinocandins, as a new class of antifungal agents, exhibit good effects in the treatment of infections caused by *Candida* or *Aspergillus.* Caspofungin and Micafungin are the representatives of such medicaments. Echinocandins inhibit fungus by suppressing the formation of 1,3-β glycosidic bond, so as to reduce the harm to human body, and reduce the side effects while remaiming high efficiency. Therefore, they are safer in use than traditional antifungal agents.

FK463 (sodium Micafungin) is the compound of formula II (R is a sodium ion), which is developed by Japan Fujisawa Toyama Co., Ltd, Takaoka Plant under the trade name Mycamine, and currently sold in several countries as antifungal agent for intravenous administration. It is obtained by cutting the side-chain of FR901379 as precursor (compound of Formula III, R is a sodium ion or a hydrogen ion) by enzyme, thus forming FR179642 (compound of Formula I, R is a hydrogen or a sodium ion) (see U.S. Patent No. US5376634, EP0431350 and Chinese patent CN1161462C for specific methods), and then chemically modifying FR179642 (see Patent Publication WO9611210, WO9857923, WO2004014879 for specific preparation and purification methods).

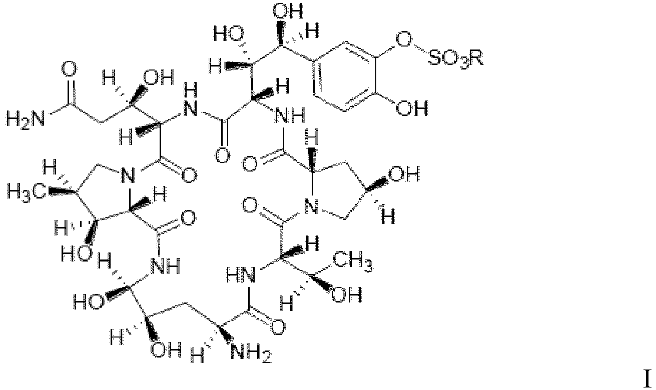

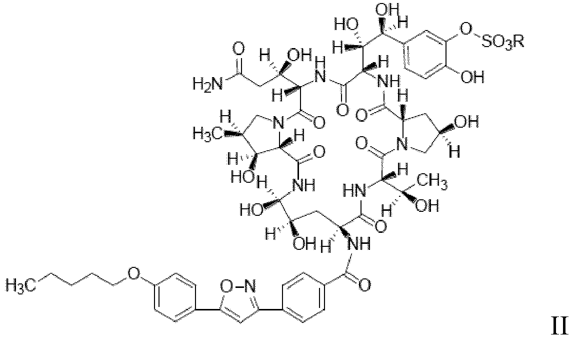

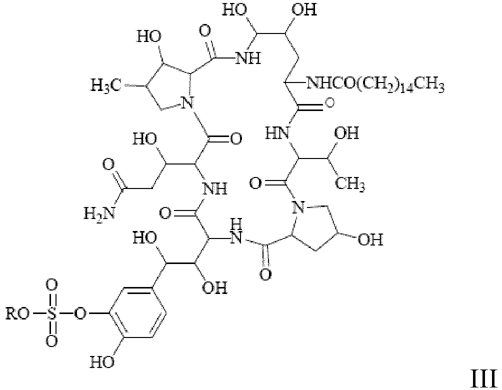

Specific scheme is shown as follows:

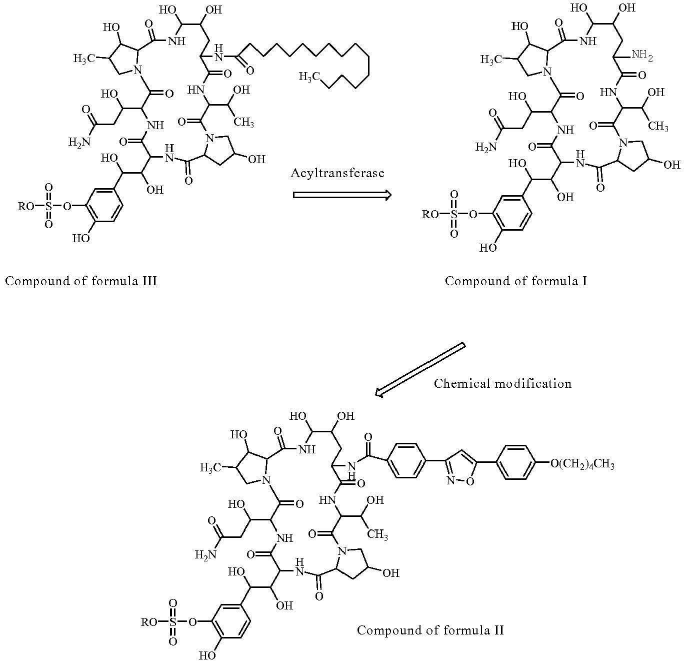

However, the present inventors have analyzed existing Micafungin formulation by HPLC, and found that impurity 6a, impurity 7a, impurity 8a, impurity 9a and impurity 10a are contained in the formulation. And the inventors have prepared impurity 6a, impurity 7a, impurity 8a, impurity 9a and impurity 10a by using preparative column in small amount, and confirmed the structure of the impurity by MS and ¹H-NMR as formula IVa, V a, VIa, VIIa, VIIIa respectively:
Impurities 6a, the chemical name of which is 5-[(1S,2S,3S)-4-[(1S,2R)-4-amino-1-[[(2S,3S,4S)-2-carbamyl-3-hydroxy-4-methyl-1-pyrrolyl]carbonyl]-2-hydroxy-4-oxobutyl]amino]-3-[[[(2S,4R)-1-[(2S,3R)-2-[[[(2S,4R )-4,5-dihydroxy-1-[4-[5-[4-(pentyloxy)phenyl]-3-isoxazolyl]benzoyl]-2-pyrrolyl]carb onyl]amino]-3-hydroxybutyryl]-4-hydroxy-2-pyrrolyl]carbonyl]amino]-1,2-dihydroxy -4-oxobutyl]-2-hydroxyphenyl sodium sulfate 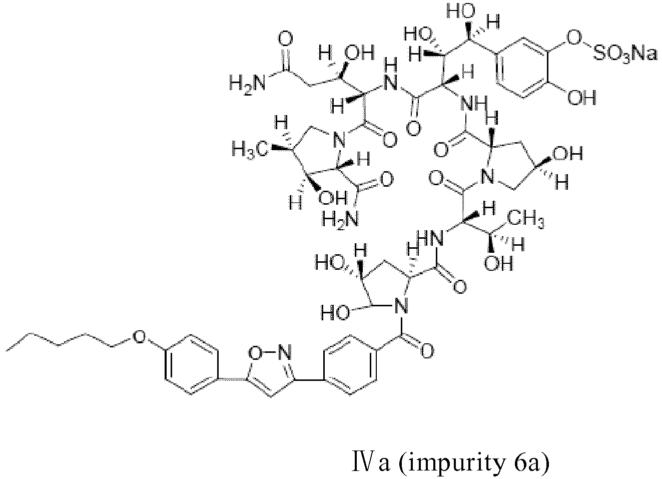 MS and ¹H-NMR data of impurity 6a are listed as follows:
   MS: 1314.5[M+Na]⁺
   ¹H-NMR(DMSO-d₆): 0.81-1.03(6H,m), 1.12(3H,d), 1.3-1.7(4H,m), 1.7-2.1(5H,m), 2.11-2.41(3H,m), 2.52-2.62(1H,m), 3.03-3.14(1H,m), 3.62-4.65(15H,m), 4.70-5.22(10H,m), 5.24(1H,d), 5.53(1H,d), 6.53-6.71(3H,m), 7.12-7.70(7H,m), 7.82(2H,d), 7.83-8.24(5H,m), 8.61-9.11(2H,m)
Impurities 7a, the chemical name of which is 5-[(1S,2S)-2-[(3S,6S,9S,11R,15S,18S,20R,21R,24S,25S)-3-[(R)-2-carbamoyl-1-hydro xyethyl]-11,20,21,25-tetrahydroxy-15-[(R)-1-hydroxyethyl]-2, 5,8,14,17,23-hexacarbo nyl-18-[4-[5-(4-pentyloxyphenyl)isoxazol-3-yl]benzamide]-1,4,7,13,16,22-hexaazatric yclo[22.3.0.0^{9,13}]heptacosan-6-yl]-1,2-dihydroxyethyl]-2-hydroxyphenyl sodium sulfate: 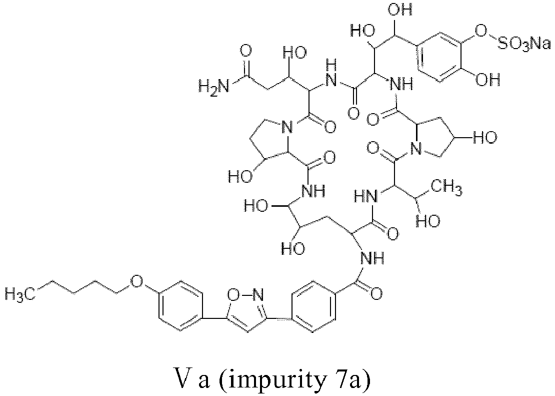 MS and ¹H-NMR data of impurity 7a are listed as follows:
   MS:1300.5[M+Na]⁺
   ¹H-NMR(DMSO-d₆): 0.87(3H,t), 1.12(3H,d), 1.42-1.65(4H,m), 1.62-2.16(6H,m), 2.11-2.43(3H,m), 2.51-2.65(1H,m), 3.04-3.11(1H,m), 3.62-4.63(15H,m), 4.71-5.23(10H,m), 5.24(1H,d), 5.69(1H,d), 6.51-6.72(3H,m), 7.11-7.74(7H,m), 7.83(2H,d), 7.84-8.11(4H,m), 8.26(1H,d), 8.61-9.12(2H,m)
Impurities 8a, the chemical name of which is 5-[(1S,2S)-2-[(3S,6S,9S,11R,15S,18S,20R,21R,24S,25S,26S)-3-[(R)-2-carbamoyl-1-11 ydroxyethyl]-11,20,21,25-tetrahydroxy-15-[(R)-1-hydroxymethyl]-26-methyl-2,5,8,14 ,17,23-hexacarbonyl-18-[4-[5-(4-pentyloxyphenyl)isoxazol-3-yl]benzamide]-1,4,7,13, 16,22-hexaazatricyclo[22.3.0.0^{9,13}]heptacosan-6-yl]-1,2-dihydroxyethyl]-2-hydroxyph enyl sodium sulfate 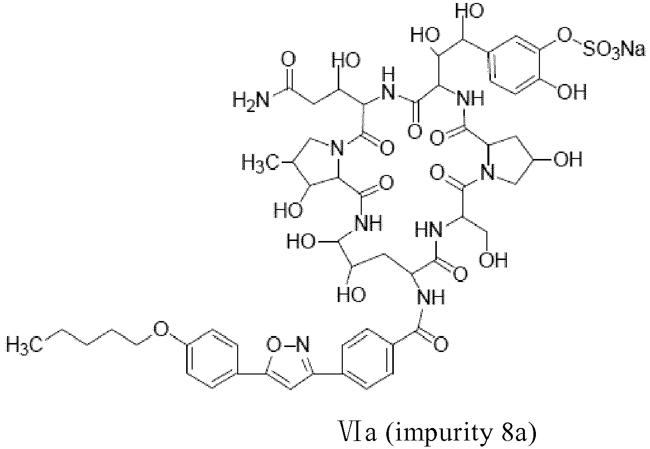 MS and ¹H-NMR data of impurity 8a are listed as follows:
   MS:1300.4[M+Na]⁺
   ¹H-NMR(DMSO-d₆): 0.83-1.02 (6H,m), 1.32-1.72(4H,m), 1.75-2.14(6H,m), 2.13-2.46(3H, m), 2.52-2.64(1H, m), 3.01-3.13(1H, m), 3.62-4.64(15H, m), 4.72-5.22(10H,m), 5.24(1H,d), 5.53(1H,d), 6.52-6.73(3H,m), 7.11-7.75(7H,m), 7.85(2H,d), 7.85-8.17(4H,m), 8.27(1H,d), 8.65-9.12(2H,m)
Impurities 9a, the chemical name of which is 5-[(1S,2S)-2-[(3S,6S,9S,11R,15S,18S,20R,21S,24S,25S,26S)-3-[(R)-2-carbamoyl-1-h ydroxyethyl]-11,20,21,25-tetrahydroxy-15-[(R)-1-hydroxyethyl]-26-methyl-2,5,8,14,1 7,23-18-hexacarbonyl-18-[4-[5-(4-pentyloxyphenyl)isoxazol-3-yl]benzamide]-1,4,7,1 3,16,22-hexaazatricyclo[22.3.0.0^{9,13}]heptacosan-6-yl]-1,2-dihydroxyethyl]-2-hydroxyp henyl sodium sulfate 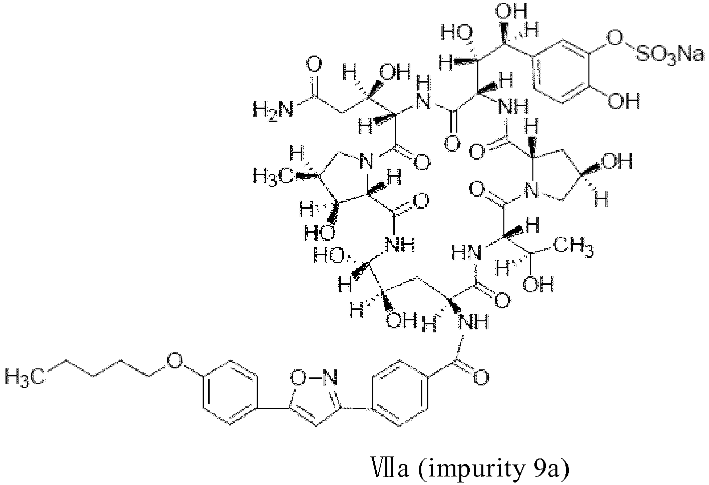 MS and ¹H-NMR data of impurity 9a are listed as follows:
   MS:1314.4[M+Na]⁺
   ¹H-NMR(DMSO-d₆): 0.87-1.11 (6H, t), 1.13(3H, d), 1.41-1.61(4H, m), 1.62-2.17(6H, m), 2.11-2.43(3H, m), 2.53-2.65(1H, m), 3.05-3.11(1H, m), 3.62-4.63(15H, m), 4.71-5.23(10H, m), 5.26(1H, d), 5.69(1H, d), 6.53-6.72(3H, m), 7.11-7.72(7H,m), 7.81(2H, d), 7.86-8.11(4H, m), 8.28(1H, d), 8.62-9.12(2H, m)
Impurities 10a, the chemical name of which is 5-[(1S,2S)-2-[(3S,6S,9S,11R,15S,18S,20R,21R,24S,25S,26S)-3-[(R)-2-carbamoyl-1-h ydroxyethyl]-11,21,25-trihydroxy-15-[(R)-1-hydroxyethyl]-26-methyl-2,5,8,14,17,23-hexacarbonyl-18-[4-[5-(4-pentyloxyphenyl)isoxazol-3-yl]benzamide]-1,4,7,13,16,22-h exaazatricyclo[22.3.0.0^{9,13}]heptacosan-6-yl]-1,2-dihydroxyethyl]-2-hydroxyphenyl sodium sulfate 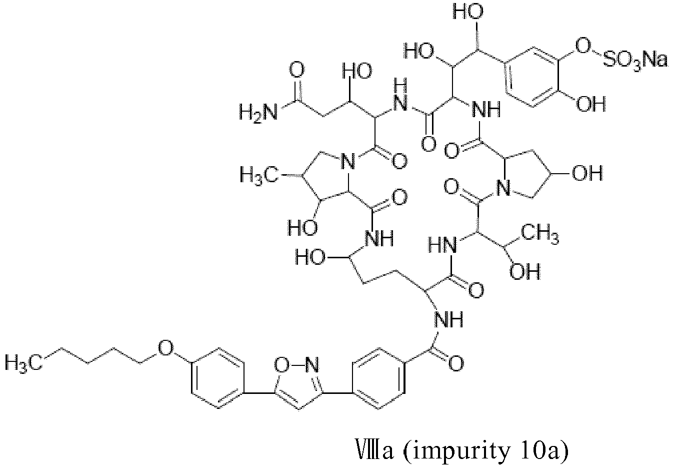 MS and ¹H-NMR data of impurity 10a are listed as follows:
   MS:1298.4[M+Na]⁺
   ¹H-NMR(DMSO-d₆): 0.87-1.11 (6H, t), 1.13(3H, d), 1.41-1.61(4H, m), 1.62-2.17(8H, m), 2.11-2.43(3H, m), 2.53-2.65(1H, m), 3.05-3.11(1H, m), 3.62-4.63(14H, m), 4.71-5.23(10H, m), 5.26(1H, d), 5.69(1H, d), 6.53-6.72(3H, m), 7.11-7.72(7H,m), 7.81(2H, d), 7.86-8.11(4H, m), 8.28(1H, d), 8.62-9.12(2H, m)

In the formulation, the amount of impurity 6a is greater than 0.3%, the total amount of impurities 7a and 8a is greater than 0.6%, the amount of impurity 9a is greater than 0.2%, the amount of impurity 10a is greater than 0.2%, and the amount of Micafungin is merely about 98.0%. However, as requested by FDA, API should contain impurities as low as possible, so as to be clinically applied safer. For example: as requested by FDA, the amount of certain impurity should be maintained below 0.1% (see ICH Good Manufacturing Practice Guide for Active Pharmaceutical Ingredients, Q7A, Current Step 4 Version (November 10, 2000) for details).

WO03018615 describes crystallization process of Micafungin sodium (e.g. compound of formula II), which can produce certain purification effects. However, the purity of commercial available Micafungin sodium formulation produced by the applicant, Japan Fujisawa Pharmaceutical Company, from Micafungin obtained by this process is merely about 98%, therefore, there is certain clinical risk for the formulation application.

As well-known in the art, the higher the purity of drug intermediates, the higher the purity of the final drug product produced through chemical modification. Similarly, the higher the purity of the compound of formula I as the intermediate, the higher the purity of the post-reaction compound of formula II (FK463) obtained by chemical modification. If the purity of the compound of formula II (FK463) obtained from chemical modification is high, the pressure on the purification of the compound of formula II will be greatly released, and the final product, the compound of formula II (FK463), with high purity can be obtained by a simple purification process. From the structure of structural analogs of micafungin, such as impurity 6, impurity 7, impurity 8, impurity 9, impurity 10, it can be determined that the above structural analogs are substantially derived from the structural analogs of compound of formula I by chemical modification reaction.

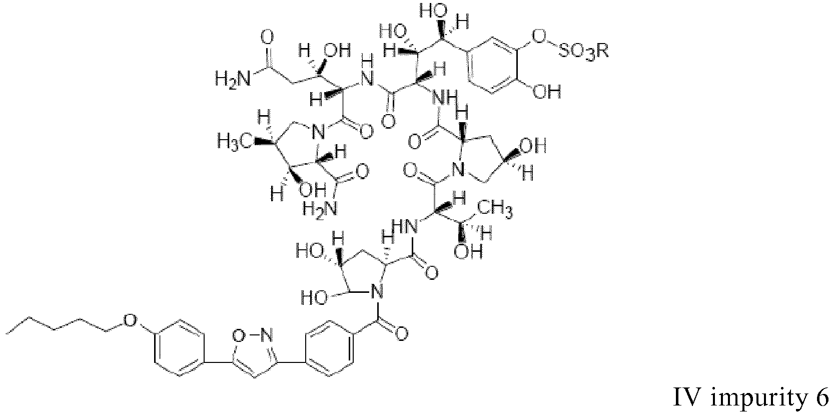

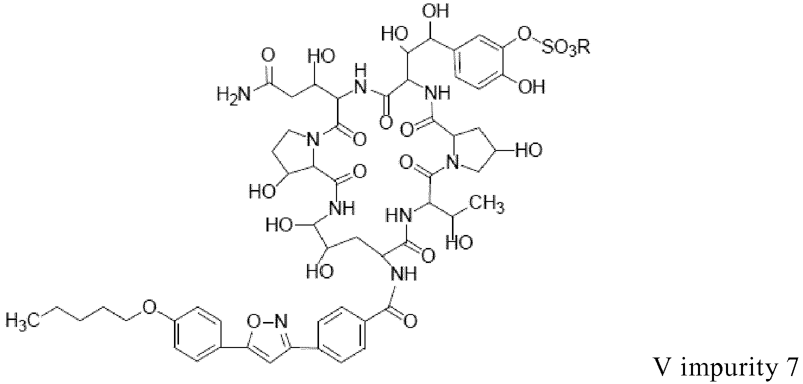

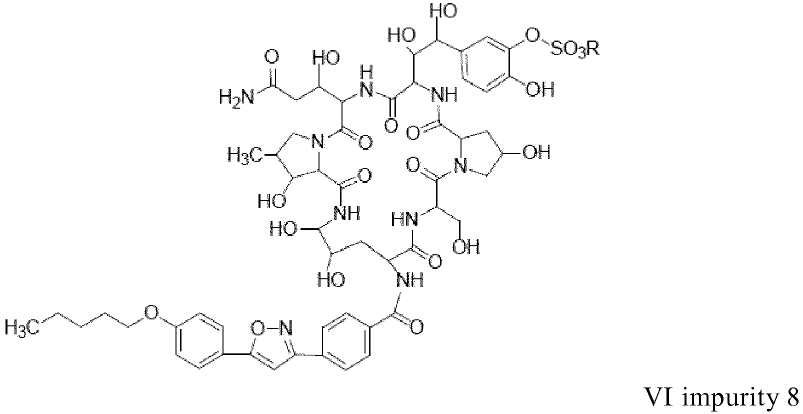

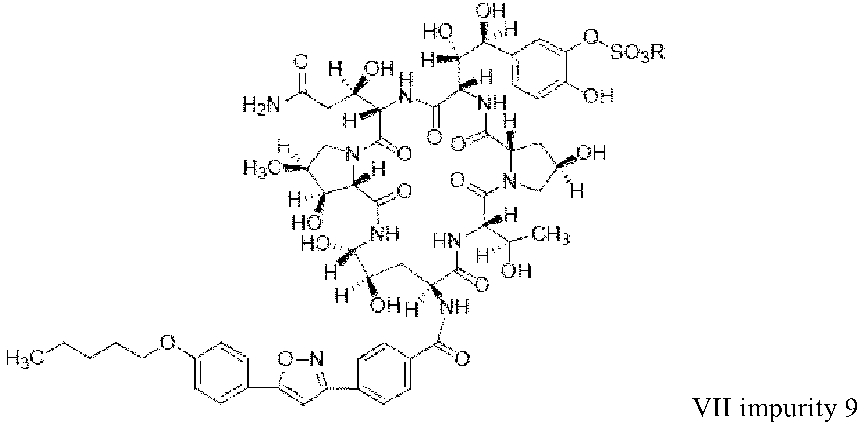

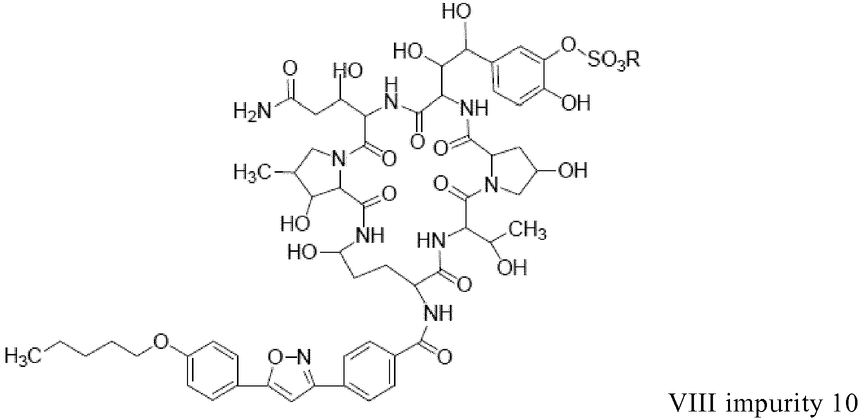

However, it is very difficult to separate structural analogs of compound of formula I, especially when taking into account the purity and yield during the purification simultaneously. CN91104847 described the purification of compound of formula I, wherein YMC GEL ODS-AM 120 is used as the filler for the major purification means, and preparative HPLC is used for purifying the compound of formula I. From formula I, it can be found that the compound of formula I possesses strong polarity and good hydrophilicity, and will be weakly retained on MC GEL ODS-AM 120 filler. Therefore, good purification effects can not be obtained by using preparative HPLC with YMC GEL ODS-AM 120 filler. The purity of compound of formula I obtained by the purification process of CN91104847 is merely 97.51 %.

Furthermore, a variety of purification process conventionally used in the art, including ion exchange resin, macroporous absorption resin, and reverse-phase preparative chromatography bonded with C18 silica gel and normal preparative chromatography with spherical silica gel, are used by the inventors to purify the compound of formula I through chromatography. However, the compound of formula I with a total purity over 99.0% can not be obtained.

By certain purification means, such as crystallization and / or recrystallization, the purity of the compound of formula I has been improved from about 97% to more than 99.0% of total purity (preferably, more than 99.8% of total purity) by the inventors, and the amount of each single impurity is less than 0.25%. And the yield during crystallization is high, which is very suitable for industrial production.

In WO9611210, WO03018615 and WO2004014879, the synthesis and purification process for Micafungin have been reported. WO9611210 reported a separation method with preparative column, however, such method requires a large amount of organic solvent, causing serious pollution to the environment, and is difficult to scale-up; and the purity of resulting product is not high. In WO03018615, WO2004014879, purification is performed by crystallization, however, impurity 6, impurity 7, impurity 8, impurity 9 and impurity 10 can not be efficiently removed by crystallization.

Therefore, the present inventors eagerly desired to obtain the high purity compound of formula I, i.e., the drug intermediate, by certain purification means in the drug intermediate stage. And then the high purity compound of formula II (FK463) is obtained by chemical modification, so as to prepare high purity Micafungin in compliance with FDA requirements.

### Summary of the Invention

One object of the present invention is to provide a substance, such as the high purity compound of formula I.

Another object of the present invention is to provide a preparation method for the high-purity substance (compound of formula I).

The third object of the present invention is to provide a use of the high-purity substance (compound of formula I).

The fourth object of the present invention is to provide another high purity substance (compounds of formula II).

The fifth object of the present invention is to provide a preparation method for another high-purity substance (compound of formula II).

### High-purity Compound of formula I

In the present invention, the high purity compound of formula I is provided, and the purity is not less than 99.0%; wherein, R represents H or a cation capable of forming a pharmaceutically acceptable salt; preferably H, a sodium ion or a diisopropylethylamine ion.

In a preferred embodiment of the invention, the purity of compound of formula I is not less than 99.2%.

In a preferred embodiment of the invention, the purity of compound of formula I is not less than 99.5%.

In another preferred embodiment of the invention, the purity of compound of formula I is not less than 99.8%.

The purity of the compound of formula I is determined by HPLC method.

The purity of the compound of formula I and / or the amount of impurities is calculated as follows: the area under curve of the peak in HPLC pattern for the compound of formula I and / or a impurity is divided by the total area under curve of HPLC pattern.

HPLC method is listed as follows:
Column: ACE 3 AQ, 150 × 4.6mm, 3µm
Mobile phase: A: 1000 ml of water, 10 ml of methanol, 100 µl of trifluoroacetic acid
B: 600 ml of water, 400ml of methanol, 100µl of trifluoroacetic acid
Flow rate: 0.55 ml/min
Column temperature: 50°C
Gradient:

| time | Mobile phase A | Mobile phase B |
|---|---|---|
| min | % | % |
| 0 | 100 | 0 |
| 25 | 100 | 0 |
| 55 | 55 | 45 |
| 56 | 0 | 100 |
| 61 | 0 | 100 |
| 62 | 100 | 0 |
| 70 | 100 | 0 |

Injector temperature: 5 °C
Detection wavelength: 225 nm

In another preferred embodiment of the present invention, the amount of impurity A in the high purity compound of formula I is not more than 0.25%.

In another preferred embodiment of the present invention, the relative retention time (abbreviated as RRT) of impurity A in HPLC is around 0.45, i.e., 0.45 ± 0.02.

In another preferred embodiment of the present invention, the amount of impurity A is not more than 0.10%.

In another preferred embodiment of the present invention, the amount of impurity A is not more than 0.05%.

In another preferred embodiment of the present invention, the amount of impurity B in the high purity compound of formula I is not more than 0.25%.

In another preferred embodiment of the present invention, the relative retention time (abbreviated as RRT) of impurity B in HPLC is around 0.65, i.e., 0.65 ± 0.02.

In another preferred embodiment of the present invention, the amount of impurity B is not more than 0.15%.

In another preferred embodiment of the present invention, the amount of impurity B is not more than 0.10%.

In another preferred embodiment of the present invention, the amount of impurity B is 0.03%-0.10%.

In another preferred embodiment of the present invention, the amount of impurity B is not more than 0.03%.

In another preferred embodiment of the present invention, the amount of impurity C in the high purity compound of formula I is not more than 0.25%.

In another preferred embodiment of the present invention, the relative retention time (abbreviated as RRT) of impurity C in HPLC is around 0.88, i.e., 0.88 ± 0.02.

In another preferred embodiment of the present invention, the amount of impurity C is not more than 0.15%.

In another preferred embodiment of the present invention, the amount of impurity C is not more than 0.10%.

In another preferred embodiment of the present invention, the amount of impurity C is 0.02%-0.10%.

In another preferred embodiment of the present invention, the amount of impurity C is not more than 0.02%.

In another preferred embodiment of the present invention, the amount of impurity D in the high purity compound of formula I is not more than 0.20%.

In another preferred embodiment of the present invention, the relative retention time (abbreviated as RRT) of impurity D in HPLC is around 1.08, i.e., 1.08 ± 0.02.

In another preferred embodiment of the present invention, the amount of impurity D is not more than 0.15%.

In another preferred embodiment of the present invention, the amount of impurity D is not more than 0.10%.

In another preferred embodiment of the present invention, the amount of impurity D is 0.04%-0.10%.

In another preferred embodiment of the present invention, the amount of impurity D is not more than 0.04%.

In another preferred embodiment of the present invention, the amount of impurity E in the high purity compound of formula I is not more than 0.15%.

In another preferred embodiment of the present invention, the relative retention time (abbreviated as RRT) of impurity E in HPLC is around 1.29, i.e., 1.29 ± 0.02.

In another preferred embodiment of the present invention, the amount of impurity E is not more than 0.10%.

In another preferred embodiment of the present invention, the amount of impurity E is not more than 0.05%.

In another preferred embodiment of the present invention, the amount of impurity F in the high purity compound of formula I is not more than 0.15%.

In another preferred embodiment of the present invention, the relative retention time (abbreviated as RRT) of impurity F in HPLC is around 1.92, i.e., 1.92 ± 0.02.

In another preferred embodiment of the present invention, the amount of impurity F is not more than 0.10%.

In another preferred embodiment of the present invention, the amount of impurity F is not more than 0.05%.

In another preferred embodiment of the present invention, wherein the amount of any other relevant impurities in the high purity compound of formula I is not more than 0.10%, and said other relevant impurities refer to impurities other than impurities A-F which may be present.

In another preferred embodiment of the present invention, the amount of any other relevant impurities is not more than 0.05%.

In another preferred embodiment of the present invention, the amount of other relevant impurities is 0%.

### Preparation of high purity compound of formula I

After study, the inventors unexpectedly discovered that crystals with excellent morphology can be formed from the compound of formula I by dissolving the compound into water or mixture solution of water-miscible lower alcohols, maintaining the solution of compound of formula I around saturated solubility and controlling pH value of the solution at specified range. Such crystallization process will produce good purification effects, thereby preparing the high purity compound of formula I. Furthermore, since the compound of formula I is cyclopeptide compound, the peptide bond formed during the condensation of amino acid will break by hydrolysis in a solution under high temperature. Therefore, the crystallization process for the compound of formula I should be controlled at certain temperature range so as to ensure that the cyclopeptide will not degrade through ring-opening. For the preparation method of the invention, a great deal of intensive researching works have been performed on screening solvents for crystallization, and it is found that crystals with excellent morphology can be formed from the compound of formula I by crystallizing the compound in methanol, ethanol, n-propanol, isopropanol or the mixture solution thereof, and the crystallization process will produce good purification effects. However, when crystallizing the compound in a solvent such as acetone, acetonitrile, ethyl acetate, the compound of formula I will form into amorphous precipitate, and purification effects, such as removal of impurities, can not be achieved by such precipitation process.

The preparation method of the present invention includes the following steps:
(a) dissolving the crude compound of formula I into water or aqueous organic solvent (i), and controlling pH of the solution;
(b) obtaining the high purity compound of formula I by reducing the temperature and / or adding organic solvent (i).

In step (a), the temperature for dissolution is 10 to 50°C, preferably 20 to 40°C.

In step (a), the volume ratio of organic solvent (i) to water in the aqueous solution of organic solvent (i) is 0.01 to 100, preferably 0.1 to 10, more preferably 0.5 to 3.0.

In step (a), the solution comprises 10 to 500 mg/ml, preferably 100 to 400 mg/ml of compound of formula I, based on the total volume of the solution in step (a).

In step (a), pH of the solution is controlled at 2.0-5.0, preferably 3.5-4.5.

In step (b), the temperature is reduced to -40 to 35°C, preferably -10 to 35°C, more preferably -5 to 30°C, and the most preferably 5 to 10°C.

In step (b), the volume ratio of organic solvent (i) to the solution of step (a) is 0.1 to 50, preferably 0.1 to 10, and the most preferably 1-5.

In step (a) and / or (b), said organic solvent (i) is a C1-C4 lower alcohol; preferably selected from: methanol, ethanol, n-propanol, isopropanol, or a mixture thereof.

The high purity compound of formula I obtained in step (b) is crystals.

After step (b), there can be the following steps:
(c) centrifuging or filtrating;
(d) removing the solvent and most of the water (drying), so as to obtain the high purity compound of Formula I.

Step (a) - (b) can be repeated for one or more times for recrystallization, preferably 1-4 times.

In one embodiment of the present invention, the high purity compound of Formula I can be obtained by using the following steps:
(a) dissolving the compound of formula I into water, and controlling pH of the solution;
(b) obtaining the crystals of compound of formula I by reducing the temperature of the solution;
(c) centrifuging or filtrating;
(d) drying, so as to obtain the high purity compound of Formula I.

In step (a), the temperature for dissolution is 10 to 50°C, preferably 20 to 40°C.

In step (a), the solution comprises 10 to 500 mg/ml, preferably 100 to 400 mg/ml of compound of formula I, based on the total volume of the solution.

In step (a), pH of the solution is controlled at 2.0-5.0, preferably 3.5-4.5.

In step (b), the temperature is reduced to -10 to 35°C, more preferably -5 to 30°C, and the most preferably 5 to 10°C.

In another embodiment of the present invention, the high purity compound of Formula I can be obtained by using the following steps:
(a) dissolving the compound of formula I into water, and controlling pH of the solution;
(b) adding organic solvent (i), so as toprecipitate out the crystals of compound of formula I completely;
(c) centrifuging or filtrating;
(d) drying the crystalls, so as to obtain the high purity compound of Formula I.

In step (a), the temperature for dissolution is 10 to 50°C, preferably 20 to 40°C.

In step (a), the solution comprises 10 to 500 mg/ml, preferably 50 to 300 mg/ml of compound of formula I, based on the total volume of the solution.

In step (a), pH of the solution is controlled at 2.0-5.0, preferably 3.5-4.5.

In step (b), said organic solvent (i) is a C1-C4 lower alcohol; preferably selected from: methanol, ethanol, n-propanol, isopropanol, or a mixture thereof.

In step (b), the volume ratio of organic solvent (i) to the solution of step (a) is 0.1 to 50, preferably 0.1 to 10, and the most preferably 1-5.

In another embodiment of the present invention, the high purity compound of Formula I can be obtained by using the following steps:
(a) dissolving the compound of formula I into water, and controlling pH of the solution;
(b) reducing the temperature of solution and adding organic solvent (i), so as to preticipate out the crystals of compound of formula I completely;
(c) centrifuging or filtrating;
(d) drying the crystalls, so as to obtain the high purity compound of Formula I.

In step (a), the temperature for dissolution is 10 to 50°C, preferably 20 to 40°C.

In step (a), the solution comprises 10 to 500 mg/ml, preferably 50 to 300 mg/ml of compound of formula I, based on the total volume of the solution.

In step (a), pH of the solution is controlled at 2.0-5.0, preferably 3.5-4.5.

In step (b), said organic solvent (i) is a C1-C4 lower alcohol; preferably selected from: methanol, ethanol, n-propanol, isopropanol, or a mixture thereof.

In step (b), the temperature is reduced to -40 to 35°C, preferably -10 to 35°C, more preferably -5 to 30°C, and the most preferably 5 to 10°C.

In step (b), the volume ratio of organic solvent (i) to the solution of step (a) is 0.1 to 50, preferably 0.1 to 10, and the most preferably 1-5.

In another embodiment of the present invention, the high purity compound of Formula I can be obtained by using the following steps:
(a) dissolving the compound of formula I into aqueous solution of organic solvent (i), and controlling pH of the solution;
(b) reducing the temperature of solution, so as to preticepate out the crystals of compound of formula I;
(c) centrifuging or filtrating;
(d) drying the crystalls, so as to obtain the high purity compound of Formula I.

In step (a), the temperature for dissolution is 10 to 50°C, preferably 20 to 40°C.

In step (a), the volume ratio of organic solvent (i) to water in the aqueous solution of organic solvent (i) is 0.01 to 100, preferably 0.1 to 10, and the most preferably 0.5-3.0.

In step (a), the solution comprises 10 to 500 mg/ml, preferably 100 to 400 mg/ml of compound of formula I, based on the total volume of the solution.

In step (a), pH of the solution is controlled at 2.0-5.0, preferably 3.5-4.5.

In step (a), said organic solvent (i) is a C1-C4 lower alcohol; preferably selected from: methanol, ethanol, n-propanol, isopropanol, or a mixture thereof.

In step (b), the temperature is reduced to -40 to 35°C, preferably -10 to 35°C, more preferably -5 to 30°C, and the most preferably 5 to 10°C.

In another embodiment of the present invention, the high purity compound of Formula I can be obtained by using the following steps:
(a) dissolving the compound of formula I into aqueous solution of organic solvent (i), and controlling pH of the solution;
(b) adding organic solvent (i), so as to preticipate out the crystals of compound of formula I;
(c) centrifuging or filtrating;
(d) drying the crystalls, so as to obtain the high purity compound of Formula I.

In step (a), the temperature for dissolution is 10 to 50°C, preferably 20 to 40°C.

In step (a), the volume ratio of organic solvent (i) to water in the aqueous solution of organic solvent (i) is 0.01 to 100, preferably 0.1 to 10, and the most preferably 0.5-3.0.

In step (a), the solution comprises 10 to 500 mg/ml, preferably 100 to 400 mg/ml of compound of formula I, based on the total volume of the solution.

In step (a), pH of the solution is controlled at 2.0-5.0, preferably 3.5-4.5.

In step (b), the volume ratio of organic solvent (i) to the solution of step (a) is 0.1 to 10, preferably 1-5.

In step (a) and (b), said organic solvent (i) is a C1-C4 lower alcohol; preferably selected from: methanol, ethanol, n-propanol, isopropanol, or a mixture thereof.

In another embodiment of the present invention, the high purity compound of Formula I can be obtained by using the following steps:
(a) dissolving the compound of formula I into aqueous solution of organic solvent (i), and controlling pH of the solution;
(b) reducing the temperature of solution and adding organic solvent (i), so as to preticipate out the crystals of compound of formula I;
(c) centrifuging or filtrating;
(d) drying the crystalls, so as to obtain the high purity compound of Formula I.

In step (a), the temperature for dissolution is 10 to 50°C, preferably 20 to 40°C.

In step (a), the volume ratio of organic solvent (i) to water in the aqueous solution of organic solvent (i) is 0.01 to 100, preferably 0.1 to 10, and the most preferably 0.5-3.0.

In step (a), the solution comprises 10 to 500 mg/ml, preferably 100 to 400 mg/ml of compound of formula I, based on the total volume of the solution.

In step (a), pH of the solution is controlled at 2.0-5.0, preferably 3.5-4.5.

In step (b), the temperature is reduced to -40 to 35°C, preferably -10 to 35°C, more preferably -5 to 30°C, and the most preferably 5 to 10°C.

In step (b), the volume ratio of organic solvent (i) to the solution of step (a) is 0.1 to 50, preferably 0.1 to 10, and the most preferably 1-5.

In step (a) and (b), said organic solvent (i) is a C1-C4 lower alcohol; preferably selected from: methanol, ethanol, n-propanol, isopropanol, or a mixture thereof.

The compound of formula I obtained by the method provided in the present invention possesses high purity; therefore, it will be better to be used for the preparation of a compound of formula II.

### High-purity Compound of formula II

In one aspect, the high purity compound of formula II is provided in the present invention, and the HPLC purity thereof is not less than 98.80%; preferably, not less than 99.0%; more preferably, not less than 99.5%; wherein, R represents H or a cation capable of forming a pharmaceutically acceptable salt; preferably H, a sodium ion or a diisopropylethylamine ion.

The purity of the compound of formula II is determined by HPLC.

The purity of the compound of formula II and / or the amount of impurities is calculated as follows: the area under curve of the peak in HPLC pattern for the compound of formula II and / or a impurity is divided by the total area under curve of HPLC pattern.

The purity of the compound of formula II in liquid phase is determined by HPLC method, and HPLC method is listed as follows:
HPLC Analytic Column: YMC-ODS 250*4.6mm, 5µm
Mobile phase: acetonitrile: phosphate buffer = 70: 45
Elution program: isocratic
Flow rate: 1.15 ml/min
Column temperature: 35°C
Detection wavelength: 210 nm
Running time: 45 min
Diluent: aqueous phosphate buffer

In one preferred embodiment of the present invention, the amount of impurity 6 (the structure of which is shown in Formula IV) in the high purity compound of formula II is less than 0.2%; preferably, less than 0.1%; more preferably, less than 0.05%; wherein, R represents H or a cation capable of forming a pharmaceutically acceptable salt; preferably H, a sodium ion or a diisopropylethylamine ion.

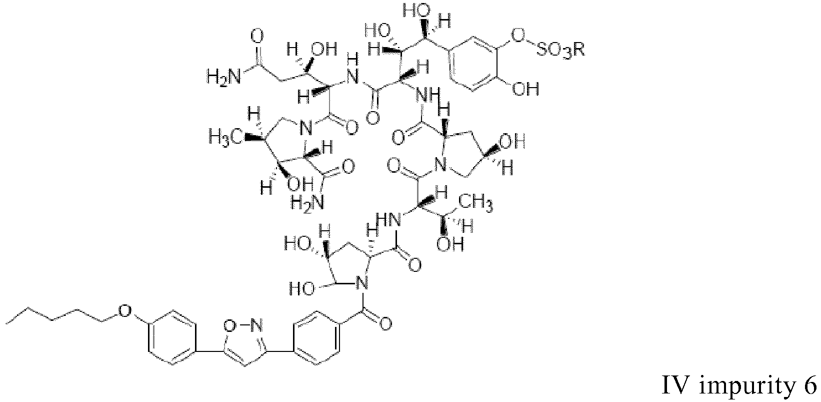

In one preferred embodiment of the present invention, the total amount of impurity 7 (the structure of which is shown in Formula V) and impurity 8 (the structure of which is shown in Formula VI) in the high purity compound of formula II is less than 0.5%; preferably, less than 0.3%; more preferably, less than 0.1%; wherein, R represents H or a cation capable of forming a pharmaceutically acceptable salt; preferably H, a sodium ion or a diisopropylethylamine ion.

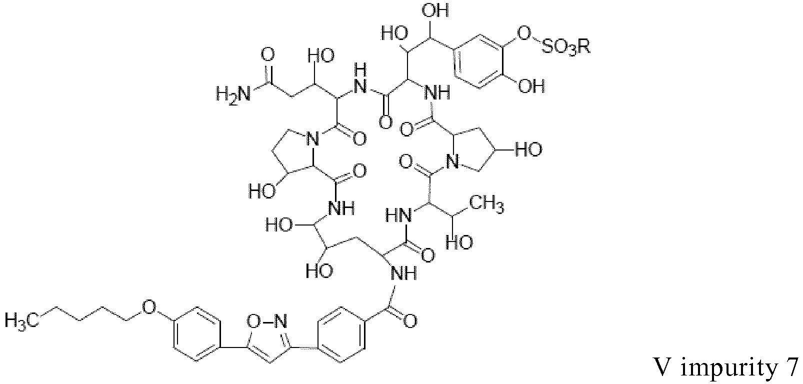

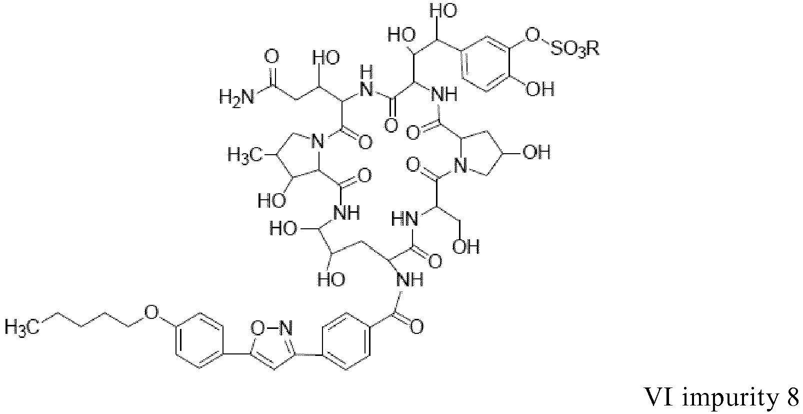

In another preferred embodiment of the present invention, the amount of impurity 9 (the structure of which is shown in Formula VII) in the high purity compound of formula II is less than 0.2%; preferably, less than 0.1%; wherein, R represents H or a cation capable of forming a pharmaceutically acceptable salt; preferably H, a sodium ion or a diisopropylethylamine ion.

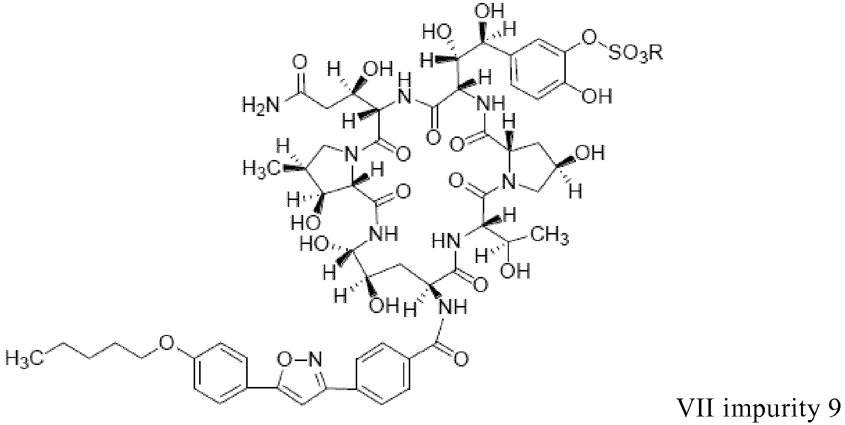

In another preferred embodiment of the present invention, the amount of impurity 10 (the structure of which is shown in Formula VIII) in the high purity compound of formula II is less than 0.2%; preferably, less than 0.1%; wherein, R represents H or a cation capable of forming a pharmaceutically acceptable salt; preferably H, a sodium ion or a diisopropylethylamine ion.

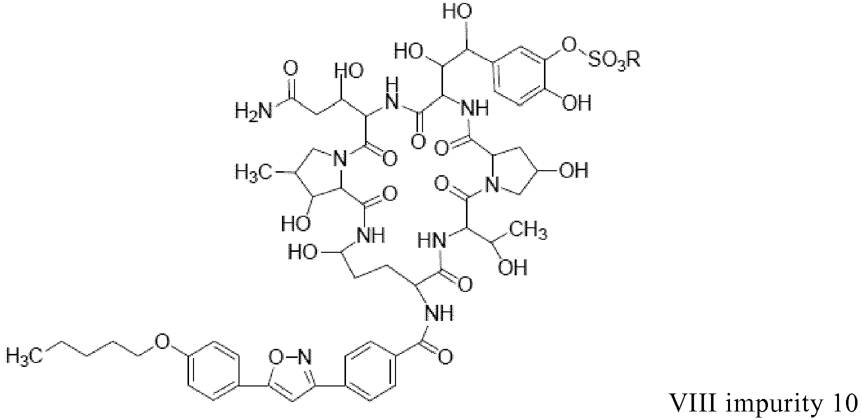

In another preferred embodiment of the present invention, the amount of impurity 11 in the high purity compound of formula II is not more than 0.15%; preferably, not more than 0.1%; the most preferably, not more than 0.05%. And the relative retention time (abbreviated as RRT) of impurity 11 in HPLC is around 0.96, i.e., 0.96 ± 0.02.

In another preferred embodiment of the present invention, wherein the amount of any other relevant impurities in the high purity compound of formula II is not more than 0.02%; preferably, not more than 0.01%; the most preferably, is 0%; and said other relevant impurities refer to impurities other than impurities 6-11 which may be present.

### The preparation method for the high-purity compound of formula II

A preparation method for the high-purity compound of formula II, wherein high purity cyclopeptide compound according to any one of claims 1-11 is used as raw material to prepare the compound of formula II; wherein, R represents H or a diisopropylethylamine ion or other cations capable of forming a pharmaceutically acceptable salt.

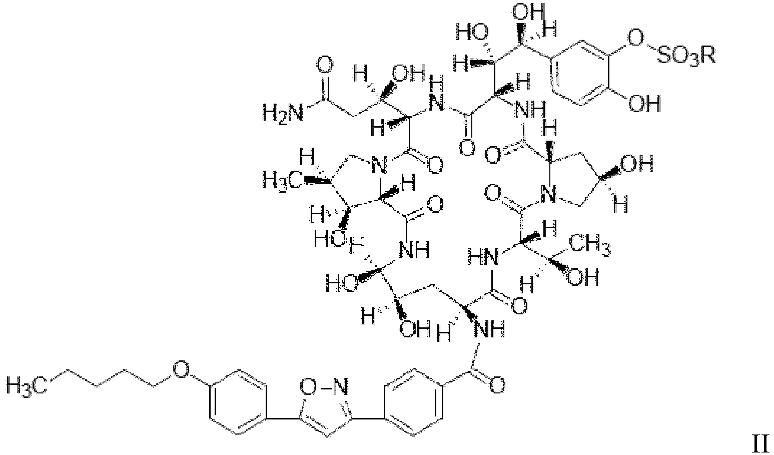

Synthetic routes of the compound have been reported in several patents, such as WO9611210, 9857923, 2604014879 etc.

### Uses of the high-purity compound of formula I and compositions comprising the same

In the present invention, uses of the high purity compound of formula I are provided. In one aspect, it can be readily used to prepare the high purity compound of formula II, wherein R represents H, diisopropylethylamine, or other cations capable of forming a pharmaceutically acceptable salt.

In another aspect, the high purity compound of formula I provided in the present invention can be directly used for preparation of medicaments for treating fungal infections. A pharmaceutical composition comprising the compound of formula I and a pharmaceutically acceptable carrier can be also provided.

### Uses of the high-purity compound of formula II and compositions comprising the same

In the present invention, uses of the high purity compound of formula II are provided. The high purity compound of formula II provided in the present invention can be directly used for preparation of medicaments for treating fungal infections. A pharmaceutical composition comprising the compound of formula II and a pharmaceutically acceptable carrier can be also provided.

In the present invention, a method for preparing a pharmaceutical composition comprising the high purity compound of formula II is provided:

The high purity compound of formula II of the present invention is mixed with a pharmaceutically acceptable carrier, so as to obtain a pharmaceutical composition comprising the high purity compound of formula II.

As used herein, "compound of formula I" or "formula I compound" may be used interchangeably, both of which refer to a compound having the following structure formula or a pharmaceutically acceptable salt thereof: 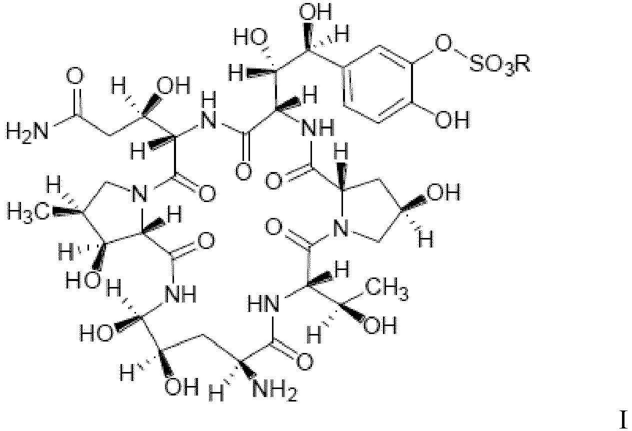 wherein, R represents H or a cation capable of forming a pharmaceutically acceptable salt, preferably H, a sodium ion or a diisopropylethylamine ion.

Preferably, pharmaceutically acceptable salts include: metal salts such as alkali metal salts (such as sodium salt, potassium salt), alkaline earth metal salts (such as calcium salt, magnesium salt, etc.), ammonium salts, salts formed with organic bases (e.g., trimethylamine salt, triethylamine salt, pyridine salt, picoline salt, dicyclohexylamine salt, N,N,-dibenzylethylenediamine salt, diisopropylethylamine salt, etc.), organic acid addition salts (such as formate, acetate, trifluoroacetate, maleate, tartrate, methanesulfonate, benzenesulfonate, toluenesulfonate, etc.), inorganic acid addition salts (e.g. hydrochloride , hydrobromide, hydroiodide, sulfate, phosphate, etc.), salts formed with an amino acid (e.g. arginine, aspartic acid, glutamic acid, etc.), and the like.

As used herein, "compound of formula II" or "formula II compound" may be used interchangeably, both of which refer to a compound having the following structure formula or a pharmaceutically acceptable salt thereof: 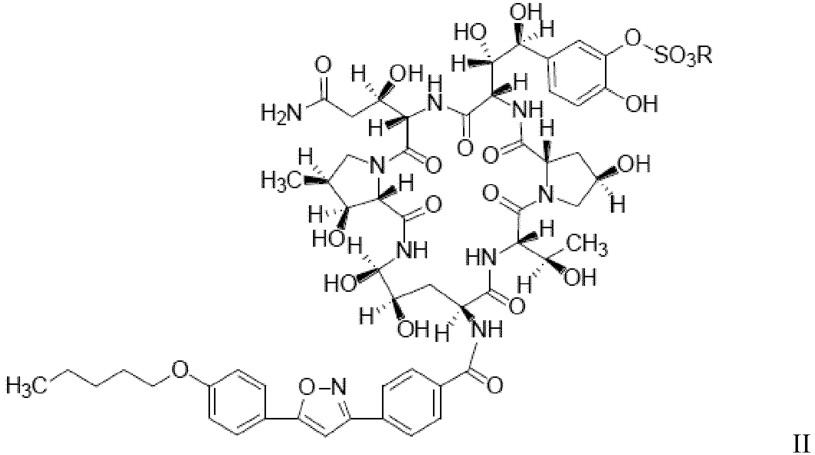 wherein, R represents H or a cation capable of forming a pharmaceutically acceptable salt, preferably H, a sodium ion or a diisopropylethylamine ion.

Preferably, pharmaceutically acceptable salts include: metal salts such as alkali metal salts (such as sodium salt, potassium salt), alkaline earth metal salts (such as calcium salt, magnesium salt, etc.), ammonium salts, salts formed with organic bases (e.g., trimethylamine salt, triethylamine salt, pyridine salt, picoline salt, dicyclohexylamine salt, N,N,-dibenzylethylenediamine salt, diisopropylethylamine salt, etc.), organic acid addition salts (such as formate, acetate, trifluoroacetate, maleate, tartrate, methanesulfonate, benzenesulfonate, toluenesulfonate, etc.), inorganic acid addition salts (e.g. hydrochloride , hydrobromide, hydroiodide, sulfate, phosphate, etc.), salts formed with an amino acid (e.g. arginine, aspartic acid, glutamic acid, etc.), and the like.

As used herein, "purity of the compound of Formula I", "purity of compound I" or " HPLC purity of compound I" may be used interchangeably, all of which refer to the percentage of measured peak area of compound I from the sum of peak area all peaks under HPLC detection conditions provided by the present invention (HPLC).

As used herein, "purity of the compound of Formula II", "purity of compound II" or " HPLC purity of compound II" may be used interchangeably, all of which refer to the percentage of measured peak area of compound II from the sum of peak area of all peaks under HPLC detection conditions provided by the present invention (HPLC).

As used herein, "crude compound of formula I" or "crude compound I" can be used interchangeably, both of which refer to a mixture, wherein the amount of compound I is < 98% under HPLC detection conditions provided in thr present invention. The crude compound I can be obtained by the methods known in the art. For example, but not limited to, the compound of formula III (a product from microorganism fermentation) as raw material is used to prepare a semi-synthetic derivative (such as the compound of formula I) through deacylation by using a deacylase, and the crude compound I is obtained after separation and purification. See US5376634, EP0431350 and CN1161462C for the method for preparing the crude compound I; and it may be obtained through commercial sources, such as, but not limited to, Fujisawa, Japan.

As used herein, the term "relative retention time (RRT)" refers to the ratio of the retention time of a peak to that of the major peak under certain HPLC condition. For example, under certain HPLC condition, if the retention time of major peak is 1 minute and the retention time of another peak is 2 minute, the relative retention time (RRT) of the latter is 2.

As used herein, the term "relative retention time (RRT)" can fluctuate within a specified range. As well-known in the art, there may be certain systematic bias for HPLC due to the adaptability of the system, resulting in a shifted relative retention time (RRT). As generally stipulated, the acceptable range is ± 0.02. For example, according to the imported drug registration standards of SFDA, there is a specified range for relative retention time (RRT) of micafungin sodium for injection and relavant impurities.

As used herein, "C1-C4 lower alcohol" refers to alcohols, the number of carbon atoms of which is 1-4.

As used herein, "pharmaceutically acceptable salts" preferably include: metal salts such as alkali metal salts (such as sodium salt, potassium salt), alkaline earth metal salts (such as calcium salt, magnesium salt, etc.), ammonium salts, salts formed with organic bases (e.g., trimethylamine salt, triethylamine salt, pyridine salt, picoline salt, dicyclohexylamine salt, N,N,-dibenzylethylenediamine salt, diisopropylethylamine salt, etc.), organic acid addition salts (such as formate, acetate, trifluoroacetate, maleate, tartrate, methanesulfonate, benzenesulfonate, toluene sulfonate, etc.), inorganic acid addition salts (e.g. hydrochloride , hydrobromide, hydroiodide, sulfate, phosphate, etc.), salts formed with an amino acid (e.g. arginine, aspartic acid, glutamic acid, etc.), and the like.

As used herein, the term "pharmaceutically acceptable carrier" refers to a carrier for administration of a therapeutic agent, including various excipients and diluents. The term refers to such carriers that they themselves are not necessary active ingredients, and won't produce undue toxicity upon administration. Suitable carriers are well-known to the skilled person in the art. In Remington's Pharmaceutical Sciences (Mack Pub. Co., NJ 1991), a full discussion on pharmaceutically acceptable excipients can be found. In the composition, pharmaceutically acceptable carriers can include liquids such as water, saline, glycerol and ethanol. Additionally, auxiliary substances may be present with these carriers, such as disintegrating agents, wetting agents, emulsifying agents, pH buffering substances and the like.

### The advantages of the invention mainly include:

1. In the present invention, the purity of the compound of formula I has been greatly improved, and the impurities have been greatly reduced, so as to obtain the high purity compound of formula I, and solve the technical problems to be solved in prior art.
2. The inventors have selected particular preparation conditions through repeated experiments, and unexpected technical effects have been produced, so that a preparation method for the high purity compound of formula I is provided, and such method is suitable for large-scale production and of high yield.
3. A novel method for preparing the high purity compound of formula II is provided in the present invention, wherein the compound of formula II can be produced from the high purity compound of formula I. The pressure on the purification of compound II will be greatly released, and the final product, the high purity compound of formula II can be obtained by a simple purification process. The yield is also greatly improved, thereby achieving unexpected technical effects.

### Brief description of the Drawings

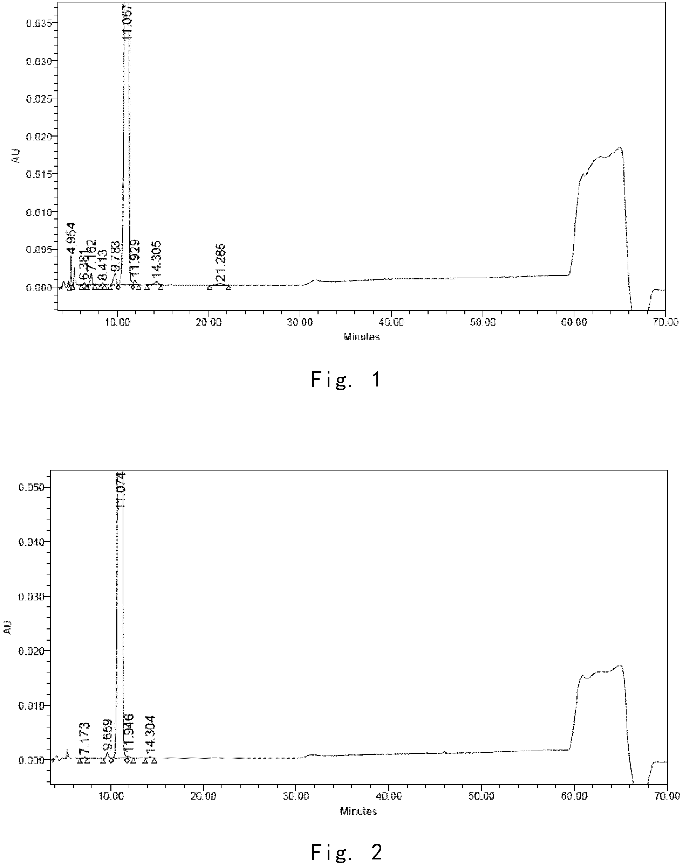

Figure 1 is the HPLC pattern of the crude compound of formula I, wherein

| | Name | Retention time (min) | Relative retention time | Peak area | Peak area% |
|---|---|---|---|---|---|
| 1 | Impurity A | 4.954 | 0.45 | 29154 | 0.42 |
| 2 | Other impurities | 6.381 | 0.58 | 5553 | 0.08 |
| 3 | Impurity B | 7.162 | 0.65 | 34014 | 0.49 |
| 4 | Other Impurities | 8.413 | 0.76 | 3470 | 0.05 |
| 5 | Impurity C | 9.783 | 0.88 | 46509 | 0.67 |
| 6 | Compound I | 11.057 | 1.00 | 6768798 | 97.51 |
| 7 | Impurity D | 11.929 | 1.08 | 19436 | 0.28 |
| 8 | Impurity E | 14.305 | 1.29 | 20824 | 0.30 |
| 9 | Impurity F | 21.285 | 1.92 | 13883 | 0.20 |

Figure 2 is the HPLC pattern of the high purity compound of formula I obtained in Example 5, wherein

| | Name | Retention time (min) | Relative retention time | Peak area | Peak area% |
|---|---|---|---|---|---|
| 1 | Impurity B | 7.173 | 0.65 | 2828 | 0.03 |
| 2 | Impurity C | 9.659 | 0.88 | 7540 | 0.08 |
| 3 | Compound I | 11.074 | 1.00 | 9406415 | 99.81 |
| 4 | Impurity D | 11.946 | 1.08 | 4716 | 0.05 |
| 5 | Impurity E | 14.304 | 1.29 | 2826 | 0.03 |

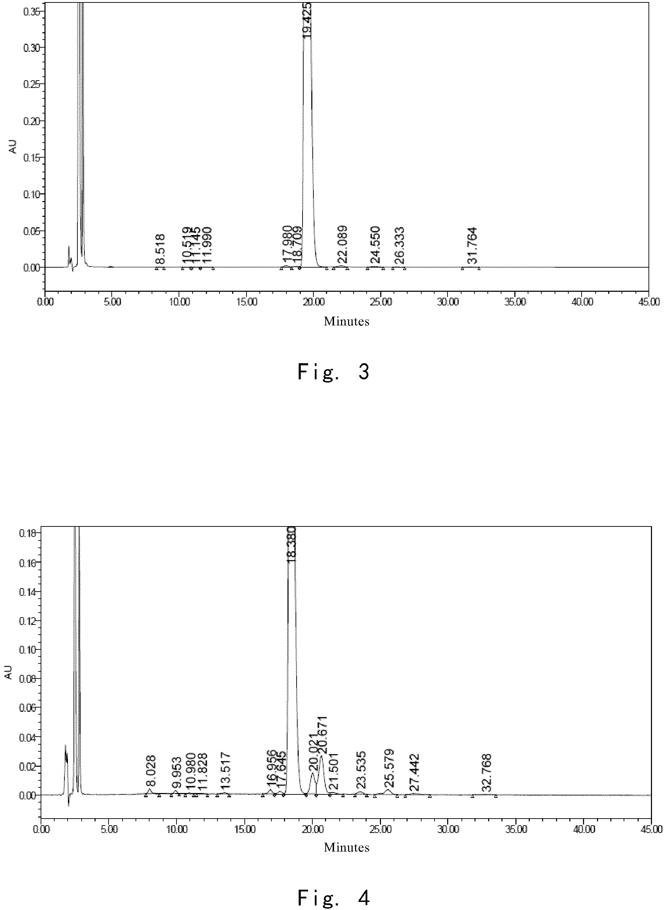

Figure 3 is the HPLC pattern of the high purity compound of formula II obtained in Example 17,

| | Retention time (min) | Relative retention time | Peak area | Peak area% |
|---|---|---|---|---|
| 1 | 8.518 | 0.44 | 8752 | 0.03 |
| 2 | 10.519 | 0.54 | 14586 | 0.05 |
| 3 | 11.145 | 0.57 | 11669 | 0.04 |
| 4 | 11.990 | 0.62 | 14597 | 0.05 |
| 5 | 17.980 | 0.92 | 49594 | 0.17 |
| 6 | 18.709 | 0.96 | 23338 | 0.08 |
| 7 | 19.425 | 1.00 | 28925210 | 99.15 |
| 8 | 22.089 | 1.13 | 61263 | 0.21 |
| 9 | 24.550 | 1.26 | 37925 | 0.13 |
| 10 | 26.333 | 1.35 | 14593 | 0.05 |
| 11 | 31.764 | 1.63 | 11637 | 0.04 |

Figure 4 is the HPLC pattern of the compound of formula II obtained in Comparative Example 3, wherein

| | Retention time (min) | Relative retention time | Peak area | Peak area% |
|---|---|---|---|---|
| 1 | 8.028 | 0.44 | 30180 | 0.21 |
| 2 | 9.953 | 0.54 | 25868 | 0.18 |
| 3 | 10.980 | 0.61 | 5748 | 0.04 |
| 4 | 11.828 | 0.64 | 4311 | 0.03 |
| 5 | 13.517 | 0.73 | 10060 | 0.07 |
| 6 | 16.956 | 0.92 | 41677 | 0.29 |
| 7 | 17.645 | 0.95 | 37365 | 0.26 |
| 8 | 18.380 | 1.00 | 13760629 | 95.75 |
| 9 | 20.021 | 1.09 | 116408 | 0.81 |
| 10 | 20.671 | 1.13 | 205511 | 1.43 |
| 11 | 21.501 | 1.17 | 24431 | 0.17 |
| 12 | 23.535 | 1.27 | 40239 | 0.28 |
| 13 | 25.579 | 1.39 | 54611 | 0.38 |
| 14 | 27.442 | 1.49 | 8622 | 0.06 |
| 15 | 32.768 | 1.78 | 5748 | 0.04 |

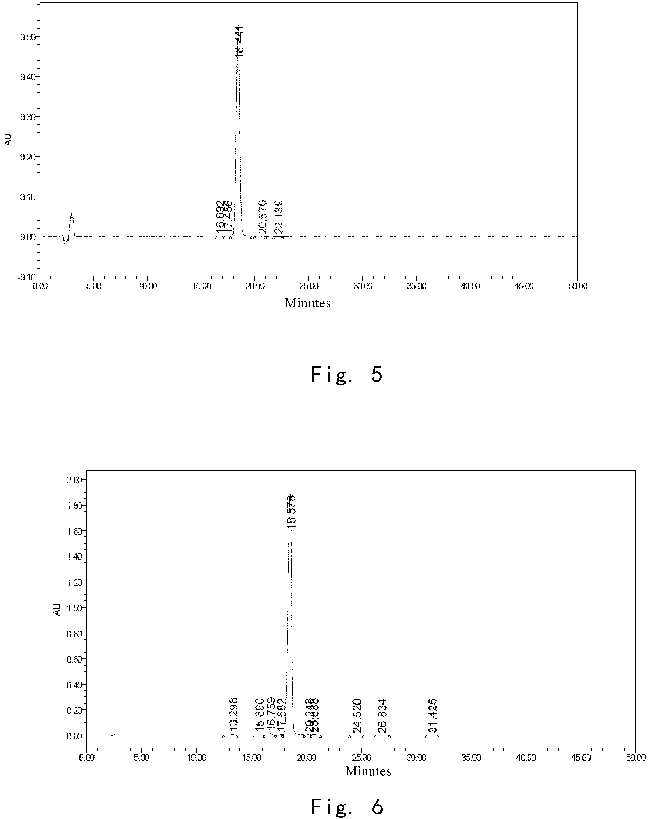

Figure 5 is the HPLC pattern of the high purity compound of formula II obtained in Example 22, wherein

| | Retention time | Relative retention time | Peak area | Peak area ratio(%) | Peak height |
|---|---|---|---|---|---|
| 1 | 16.692 | 0.91 | 10836 | 0.09 | 579 |
| 2 | 17.456 | 0.95 | 4044 | 0.03 | 244 |
| 3 | 18.441 | 1.0 | 11541182 | 99.79 | 531701 |
| 4 | 20.670 | 1.12 | 6107 | 0.05 | 250 |
| 5 | 22.139 | 1.20 | 4258 | 0.04 | 184 |

Figure 6 is the HPLC pattern of the commercially-available Micafungin sodium formulation in Comparative Example 8, wherein

| | Retention time | Relative retention time | Peak area | Peak area ratio(%) | Peak height |
|---|---|---|---|---|---|
| 1 | 13.298 | 0.72 | 152314 | 0.34 | 7118 |
| 2 | 15.690 | 0.84 | 47990 | 0.11 | 2493 |
| 3 | 16.759 | 0.90 | 318200 | 0.71 | 14054 |
| 4 | 17.682 | 0.95 | 80605 | 0.18 | 3880 |
| 5 | 18.578 | 1.0 | 43831048 | 98.01 | 1881149 |
| 6 | 20.248 | 1.09 | 125658 | 0.28 | 4405 |
| 7 | 20.688 | 1.11 | 94283 | 0.21 | 3923 |
| 8 | 24.520 | 1.32 | 28024 | 0.06 | 1075 |
| 9 | 26.834 | 1.44 | 32031 | 0.07 | 1080 |
| 10 | 31.425 | 1.69 | 10991 | 0.02 | 331 |

### The mode for carrying out the invention

The invention will be further illustrated with reference to the following specific examples. It is to be understood that these examples are only intended to illustrate the invention, but not to limit the scope of the invention. For the experimental methods in the following examples without particular conditions, they are performed under routine conditions or as instructed by the manufacturer. Unless otherwise specified, all percentages, ratios, proportions or parts are by weight.

The unit of the weight/volume percentages in the invention is well known to the skilled in the art, for example, the weight of a solute in a 100 mL solution.

Unless otherwise defined, all scientific and technical terms used herein have the same meaning as commonly understood by the skilled in the art. Furthermore, any process or material similar or equivalent to those described herein can be used in the process of the present invention. The preferred embodiments and materials described herein are merely provided for illustration.

The purity of the compound of formula I in liquid phase is determined by HPLC method, and HPLC method is listed as follows:
Column: ACE 3 AQ, 150 × 4.6mm, 3µm
Mobile phase: A: 1000 ml of water, 10 ml of methanol, 100 µl of trifluoroacetic acid
B: 600 ml of water, 400ml of methanol, 100µl of trifluoroacetic acid
Flow rate: 0.55 ml/min
Column temperature: 50°C
Gradient

| time | Mobile phase A | Mobile phase B |
|---|---|---|
| min | % | % |
| 0 | 100 | 0 |
| 25 | 100 | 0 |
| 55 | 55 | 45 |
| 56 | 0 | 100 |
| 61 | 0 | 100 |
| 62 | 100 | 0 |
| 70 | 100 | 0 |

Injector temperature: 5 °C
Detection wavelength: 225 nm

The purity of the compound of formula II in liquid phase is determined by HPLC method, and HPLC method is listed as follows:
HPLC Analytic Column: YMC-ODS 250*4.6mm, 5µm
Mobile phase: acetonitrile: phosphate buffer = 70: 45
Elution program: isocratic
Flow rate: 1.15 ml/min
Column temperature: 35°C
Detection wavelength: 210 nm
Running time: 45 min
Diluent: aqueous phosphate buffer

Wherein, the relative retention times of the compound of formula II and relevant impurities are

| name | relative retention time |
|---|---|
| Impurity 6 | 0.71-0.74 |
| Impurity 7 | 0.90-0.93 |
| Impurity 8 | 0.90-0.93 |
| The compound of formula II | 1.00 |
| Impurity 9 | 1.08-1.10 |
| Impurity 10 | 1.11-1.13 |
| Impurity 11 | 0.94-0.98 |

### Example 1

### Preparation of crude compound of formula I

76 g of the compound of formula I in solid powder was prepared according to the method of Example 1 in U.S. Patent No. 5,376,634, and the amount thereof is determined as 97.51 % by HPLC (see Fig. 1 for HPLC pattern)

**Table 1**

| Name of impurity | impurity A | impurity B | impurity C | impurity D | impurity E | impurity F |
|---|---|---|---|---|---|---|
| relative retention time | 0.45 | 0.65 | 0.88 | 1.08 | 1.29 | 1.92 |
| amount% | 0.42 | 0.49 | 0.67 | 0.28 | 0.30 | 0.20 |

### Example 2

### Preparation of the high purity compound of formula I

At 30°C, 3.6 g of the crude compound I prepared in Example 1 was dissolved into a mixture solution consisting of 25 ml of water and 20 ml of n-propanol, and stirred to completely dissolve the compound I. pH was adjusted to 3.5 using glacial acetic acid, and the solution was cooled to 15°C gradually. Crystals of compound I precipitated, and the system was stirred for 5 hours at 15°C, so that the crystals of compound I gradually grew. 90 ml of n-propanol was added dropwise. Upon addition, the resulting mixture was stirred for 1 hour at 15°C. The crystals was obtained by filtration, and dried in vacuo to give 3.5 g of compound I, the purity of which was determined by HPLC as 99.00%. The amount for main relevant impurities is shown in Table 2.

**Table 2**

| Name of impurity | impurity A | impurity B | impurity C | impurity D | Impurity E | Impurity F |
|---|---|---|---|---|---|---|
| relative retention time | 0.45 | 0.65 | 0.88 | 1.08 | 1.29 | 1.92 |
| amount% | 0.16 | 0.22 | 0.20 | 0.19 | 0.13 | 0.04 |

### Example 3

### Preparation of the high purity compound of formula I

At 40°C, 3.5 g of the compound I prepared in Example 2 (HPLC purity of which was 99.00%) was dissolved into a mixture solution consisting of 19 ml of water and 16 ml of n-propanol, and stirred to completely dissolve the compound I. pH was adjusted to 2.0 using glacial acetic acid, and the solution was cooled to 15°C gradually. Crystals of compound I precipitated, and the system was stirred for 5 hours at 15°C, so that the crystals of compound I gradually grew. 70 ml of n-propanol was added dropwise. Upon addition, the resulting mixture was stirred for 1 hour at 15°C. The crystals was obtained by filtration, and dried in vacuo to give 3.4 g of compound I, the purity of which was determined by HPLC as 99.23%. The amount for main relevant impurities is shown in Table 3.

**Table 3**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.12 | 0.19 | 0.17 | 0.16 | 0.10 | 0.03 |

### Example 4

### Preparation of the high purity compound of formula I

At 40°C, compound I prepared in Example 3 (HPLC purity of which was 99.23%) was dissolved into a mixture solution, pH of which was adjusted to 5.0 using glacial acetic acid, consisting of 8 ml of water and 7 ml of n-propanol, and stirred to completely dissolve the compound I. The solution was cooled to 15°C. Crystals of compound I precipitated, and the system was stirred for 5 hours at 15°C, so that the crystals of compound I gradually grew. 30 ml of n-propanol was added dropwise. Upon addition, the resulting mixture was stirred for 1 hour at 15°C. The crystals was obtained by filtration, and dried in vacuo to give 3.3 g of compound I, the purity of which was determined by HPLC as 99.57%. The amount for main relevant impurities is shown in Table 4.

**Table 4.**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.06 | 0.13 | 0.06 | 0.10 | 0.05 | 0.03 |

### Example 5

### Preparation of the high purity compound of formula I

At 40°C, compound I prepared in Example 4 (HPLC purity of which was 99.57%) was dissolved into a mixture solution, pH of which was adjusted to 4.0 using glacial acetic acid, consisting of 8 ml of water and 7 ml of n-propanol, and stirred to completely dissolve the compound I. The solution was cooled to 15°C. Crystals of compound I precipitated, and the system was stirred for 5 hours at 15°C, so that the crystals of compound I gradually grew. 30 ml of n-propanol was added dropwise. Upon addition, the resulting mixture was stirred for 1 hour at 15°C. The crystals was obtained by filtration, and dried in vacuo to give 3.2 g of compound I, the purity of which was determined by HPLC as 99.81 %. The amount for relevant impurities is shown in Table 5 and HPLC pattern in Fig. 2.

**Table 5**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0 | 0.03 | 0.08 | 0.05 | 0.03 | 0 |

### Example 6

### Preparation of the high purity compound of formula I

At 30°C, 2.6 g of the compound I prepared in Example 1 was dissolved into 8 ml of water, pH was adjusted to 3.8 using glacial acetic acid, and stirred to completely dissolve the compound I. 10 ml of n-propanol was slowly added dropwise. Crystals of compound I precipitated, and the system was stirred for 2 hours at 15°C. And then 35 ml of n-propanol was slowly added dropwise. Upon addition, the solution comprising compound I was slowly cooled to 15°C, and stirred for another 2 hours, for precipitating crystals of compound I completely. The wet solid of compound I was obtained by filtration. At 30°C, 8 ml of water, the pH of which was adjusted to 3.8 using glacial acetic acid, was used to dissolve the wet solid of compound I, and stirred to completely dissolve the compound I. The solution was cooled to 15°C, and 10 ml of n-propanol was slowly added dropwise for precipitating crystals of compound I. The system was stirred for another 2 hours at 15°C. 35 ml of n-propanol was slowly added dropwise. Upon addition, the system was stirred for another 2 hours for precipitating crystals of compound I completely. The wet solid of compound I was obtained by filtration. The above crystallization procedure was repeated. Upon recrystallization for 3 times, the compound I was obtained by filtration and dried in vacuo to give 2.4 g of compound I. The total yield is 92.3%, and the purity of compound I was determined by HPLC as 99.81 %.

### Example 7

### Preparation of the high purity compound of formula I

At 30°C, 1.6 g of the compound I prepared in Example 1 was dissolved into a mixture solution consisting of 80 ml of water and 80 ml of ethanol, and stirred to completely dissolve the compound I. pH was adjusted to 2.8 using glacial acetic acid, and the solution was slowly cooled to -20°C. The solution was stirred at -20°C for 2 hours. 450 ml of ethanol was slowly added dropwise. Upon addition, the solution was stirred for another 2 hours for precipitating crystals of compound I completely. The crystals was obtained by filtration, and dried in vacuo to give 1.5 g of compound I, the purity of which was determined by HPLC as 98.89%. Relevant impurities are shown in Table 6.

**Table 6**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.24 | 0.23 | 0.21 | 0.19 | 0.14 | 0.10 |

### Example 8

### Preparation of the high purity compound of formula I

At 30°C, 1.5 g of the compound I prepared in Example 7 was dissolved into a mixture solution consisting of 80 ml of water and 80 ml of ethanol, and stirred to completely dissolve the compound I. pH was adjusted to 3.0 using glacial acetic acid, and the solution was slowly cooled to -20°C. The solution was stirred at -20°C for 2 hours. 450 ml of ethanol was slowly added dropwise. Upon addition, the solution was stirred for another 2 hours for precipitating crystals of compound I completely. The crystals was obtained by filtration, and dried in vacuo to give 1.4 g of compound I, the purity of which was determined by HPLC as 99.36%. Relevant impurities are shown in Table 7.

**Table 7**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.16 | 0.12 | 0.12 | 0.13 | 0.06 | 0.05 |

### Example 9

### Preparation of the high purity compound of formula I

At 30°C, 1.4 g of the compound I prepared in Example 8 was dissolved into a mixture solution consisting of 80 ml of water and 80 ml of ethanol, and stirred to completely dissolve the compound I. pH was adjusted to 3.0 using glacial acetic acid, and the solution was slowly cooled to -20°C. The solution was stirred at -20°C for 2 hours. 450 ml of ethanol was slowly added dropwise. Upon addition, the solution was stirred for another 2 hours for precipitating crystals of compound I completely. The crystals was obtained by filtration, and dried in vacuo to give 1.3 g of compound I, the purity of which was determined by HPLC as 99.68%. Relevant impurities are shown in Table 8.

**Table 8**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.10 | 0.04 | 0.10 | 0.04 | 0.02 | 0.02 |

### Example 10

### Preparation of the high purity compound of formula I

At 10°C, 1.8 g of the compound I prepared in Example 1 was dissolved into 10 ml of water, and stirred to completely dissolve the compound I. pH was adjusted to 3.5 using glacial acetic acid, and the solution was cooled to 2°C. The solution was stirred at 2°C for 2 hours. 40 ml of isopropanol was slowly added dropwise. Upon addition, the solution was stirred for another 2 hours for precipitating crystals of compound I completely. The crystals was obtained by filtration, and dried in vacuo to give 1.5 g of compound I, the purity of which was determined by HPLC as 98.89%. The amount of relevant impurities is shown in Table 9.

**Table 9**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.20 | 0.24 | 0.23 | 0.15 | 0.14 | 0.15 |

### Example 11

### Preparation of the high purity compound of formula I

At 10°C, 1.5 g of the compound I prepared in Example 10 was dissolved into 10 ml of water, and stirred to completely dissolve the compound I. pH was adjusted to 3.5 using glacial acetic acid, and the solution was cooled to 2°C. The solution was stirred at 2°C for 2 hours. 40 ml of isopropanol was slowly added dropwise. Upon addition, the solution was stirred for another 2 hours for precipitating crystals of compound I completely. The crystals was obtained by filtration, and dried in vacuo to give 1.4 g of compound I, the purity of which was determined by HPLC as 99.41%. The amount of relevant impurities is shown in Table 10.

**Table 10**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.09 | 0.15 | 0.14 | 0.09 | 0.06 | 0.06 |

### Example 12

### Preparation of the high purity compound of formula I

At 10°C, 1.4 g of the compound I prepared in Example 11 was dissolved into 10 ml of water, and stirred to completely dissolve the compound I. pH was adjusted to 3.5 using glacial acetic acid, and the solution was cooled to 2°C. The solution was stirred at 2°C for 2 hours. 40 ml of isopropanol was slowly added dropwise. Upon addition, the solution was stirred for another 2 hours for precipitating crystals of compound I completely. The crystals was obtained by filtration, and dried in vacuo to give 1.3 g of compound I, the purity of which was determined by HPLC as 99.73%. The amount of relevant impurities is shown in Table 11.

**Table 11**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.03 | 0.07 | 0.06 | 0.07 | 0.02 | 0.02 |

### Example 13

### Preparation of the high purity compound of formula I

At 10°C, 1.3 g of the compound I prepared in Example 12 was dissolved into 10 ml of water, and stirred to completely dissolve the compound I. pH was adjusted to 3.5 using glacial acetic acid, and the solution was cooled to 2°C. The solution was stirred at 2°C for 2 hours. 40 ml of isopropanol was slowly added dropwise. Upon addition, the solution was stirred for another 2 hours for precipitating crystals of compound I completely. The crystals was obtained by filtration, and dried in vacuo to give 1.2 g of compound I, the purity of which was determined by HPLC as 99.90%. The amount of relevant impurities is shown in Table 12.

**Table 12**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.0 | 0.04 | 0.02 | 0.04 | 0.0 | 0.0 |

### Example 14

### Preparation of the high purity compound of formula I

At 20°C, 2.0 g of the compound I prepared in Example 1 was dissolved into a mixture solution consisting of 5 ml of water and 15 ml of methanol, and stirred to completely dissolve the compound I. The pH was adjusted to 4.5 using glacial acetic acid, and the solution was cooled to 10°C for precipitating crystals of compound I. And then the system was slowly cooled to -40°C, and stirred at -40°C for another 2 hours, for precipitating crystals of compound I completely. 80ml of methanol was slowly added dropwise. Upon addition, the solution was stirred for another 2 hours for precipitating crystals of compound I completely. The wet solid of compound I was obtained by filtration. At 20°C, a mixture solution consisting of 5 ml of water and 16 ml of methanol, the pH of which was adjusted to 4.5 using glacial acetic acid, was used to dissolve the wet solid of compound I, and stirred for 30 mins to completely dissolve the compound I. The solution was cooled to 10°C, and crystals of compound I precipitated. And then the system was slowly cooled to -40°C, and stirred at -40°C for another 2 hours. 80 ml of methanol was slowly added dropwise. Upon addition, the system was stirred for another 2 hours for precipitating crystals of compound I completely. The wet solid of compound I was obtained by filtration. The above crystallization procedure was repeated. Upon recrystallization for 3 times, the compound I was obtained by filtration and dried in vacuo to give 1.5 g of compound I solid. The total yield is 89.0%, and the purity of compound I was determined by HPLC as 99.83%. The amount of relevant impurities is shown in Table 13.

**Table 13**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.01 | 0.03 | 0.02 | 0.10 | 0.01 | 0 |

### Example 15

### Preparation of the high purity compound of formula I

At 50°C, 5.0 g of the compound I prepared in Example 1 was dissolved into 10 ml of water, and stirred to completely dissolve the compound I. The pH was adjusted to 5.0 using glacial acetic acid, and the solution was cooled to 30°C for precipitating crystals of compound I. And then the system was slowly cooled to 2°C, and stirred at 2°C for another 10 hours. The wet solid of compound I was obtained by filtration. At 50°C, 9 ml of water, the pH of which was adjusted to 5.0 using glacial acetic acid, was used to dissolve the wet solid of compound I, and stirred for 30 mins to completely dissolve the compound I. The solution was cooled to 30°C, and crystals of compound I precipitated. And then the system was slowly cooled to 2°C, and stirred at 2°C for another 10 hours. The wet solid of compound I was obtained by filtration. The above crystallization procedure was repeated. Upon recrystallization for 3 times, the compound I was obtained by filtration and dried in vacuo to give 3.0 g of compound I. The total yield is 85.0%, and the purity of compound I was determined by HPLC as 99.85%. The amount of relevant impurities is shown in Table 14.

**Table 14**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.02 | 0.05 | 0.01 | 0.01 | 0.02 | 0.04 |

### Comparative Example 1

### Effects of pH on the preparation of high purity compound I

At 40°C, 1.8 g of the compound I prepared in Example 1 was dissolved into a mixture solution consisting of 8 ml of water and 6 ml of isopropanol, and stirred to completely dissolve the compound I. The pH was adjusted to 6.5 using glacial acetic acid, and the solution was cooled to 20°C for precipitating crystals of compound I. The system was stirred at 20°C for 2 hours. 35 ml of isopropanol was slowly added dropwise. Upon addition, the solution was stirred for another 2 hours for precipitating crystals of compound I completely. The wet solid of compound I was obtained by filtration. At 40°C, a mixture solution consisting of 8 ml of water and 6 ml of isopropanol, the pH of which was adjusted to 6.5 using glacial acetic acid, was used to dissolve the wet solid of compound I, and stirred to completely dissolve the compound I. The solution was cooled to 20°C, and crystals of compound I precipitated. The system was stirred at 20°C for another 2 hours. 35 ml of isopropanol was slowly added dropwise. Upon addition, the solution was stirred for another 2 hours for precipitating crystals of compound I completely. The wet solid of compound I was obtained by filtration. The above crystallization procedure was repeated. Upon recrystallization for 4 times, the compound I was obtained by filtration and dried in vacuo to give 1.5 g of compound I. The total yield is 83.6%, and the purity of compound I was determined by HPLC as 98.90%. The amount of relevant impurities is shown in Table 15.

**Table 15**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.07 | 0.27 | 0.03 | 0.23 | 0.30 | 0.20 |

### Comparative Example 2

### Effects of solvents on the preparation of high purity compound I

At 30°C, 2.4 g of the compound I prepared in Example 1 was dissolved into 7 ml of water, pH was adjusted to 3.8 using glacial acetic acid, and the resulting mixture was stirred to completely dissolve the compound I. 15 ml of acetonitrile was slowly added, and stirred for 2 hours, and solids were precipitated. The microstructure of solides was observed under a microscope and found that almost all of the solids were irregular solids. The compound I was obtained by filtration, and dried in vacuo, the purity of which was determined by HPLC as 97.57%. The amount of major relevant impurities is shown in Table 16, which is scarcely changed.

**Table 16**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.42 | 0.48 | 0.64 | 0.26 | 0.40 | 0.23 |

At 18°C, 2.1 g of the compound I prepared in Example 1 was dissolved into 7 ml of water, pH was adjusted to 3.8 using glacial acetic acid, and the resulting mixture was stirred to completely dissolve the compound I. 20 ml of acetone was slowly added, and stirred for 2 hours, and solids were precipitated. The microstructure of solides was observed under a microscope and found that almost all of the solids were irregular solids. The compound I was obtained by filtration, and dried in vacuo, the purity of which was determined by HPLC as 97.79%. The amount of major relevant impurities is shown in Table 17, which is scarcely changed.

**Table 17**

| Name of impurity | Impurity A | Impurity B | Impurity C | Impurity D | Impurity E | Impurity F |
|---|---|---|---|---|---|---|
| relative retention time | 0.45 | 0.65 | 0.88 | 1.08 | 1.29 | 1.92 |
| amount% | 0.40 | 0.41 | 0.60 | 0.30 | 0.31 | 0.19 |

### Example 16

### Preparation of the high purity compound of formula II from the high purity compound of formula I

The compound of formula II was synthesized from the compound of formula I according to the process for Micafungin synthesis in WO2004014879.

The compound of formula I obtained in Example 2 of the present application (HPLC purity of which was 99.00% (1.00 g, 1.07 mmol)) was dissolved in 12 ml of DMF. The resulting solution was cooled to below 0°C in an ice bath. Diisopropylethylamine (0.22 g, 1.67 mmol) was added, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into the reaction liquid at the end of the reaction, stirred for another 1 hour, and filtered, so as to give Micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity of Micafungin diisopropylethylamine was determined as 98.81 % by HPLC, and the yield was 90.6%. Chromatographic peaks before 5 mins in HPLC Pattern corresponded to solvent and reaction by-product, 1-hydroxybenzotriazole (HOBT), which can be readily removed by the purification process reported in WO2004014879. The amount of main relevant impurities is shown in Table 18.

**Table 18**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.92 | 0.96 | 1.09 |
| amount% | 0.02 | 0.03 | 0.04 | 0.03 | 0.21 | 0.20 | 0.08 | 0.13 |

| Impurity | Impurity 10 | | Unknown | Unknown | Unknown | | | |
|---|---|---|---|---|---|---|---|---|
| relative retention time | 1.13 | | 1.26 | 1.35 | 1.63 | | | |
| amount% | 0.23 | 0.13 | 0.05 | 0.04 | | | | |

### Example 17

### Preparation of the high purity compound of formula II from the high purity compound of formula I

The compound of formula II was synthesized from the compound of formula I according to the process for Micafungin synthesis in WO2004014879.

The compound of formula I obtained in Example 3 of the present application (HPLC purity of which was 99.23% (1.00 g, 1.07 mmol)) was dissolved in 12 ml of DMF. The resulting solution was cooled to below 0°C in an ice bath. Diisopropylethylamine (0.22 g, 1.67 mmol) was added, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into the reaction liquid at the end of the reaction, stirred for another 1 hour, and filtered, so as to give Micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity of Micafungin diisopropylethylamine was determined as 99.15% by HPLC, and the yield was 91.6%. Chromatographic peaks before 5 mins in HPLC Pattern (see Fig. 3) corresponded to solvent and reaction by-product, 1-hydroxybenzotriazole (HOBT), which can be readily removed by the purification process reported in WO2004014879. The amount of main relevant impurities is shown in Table 19.

**Table 19**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.92 | 0.96 | 1.09 |
| amount% | 0.03 | 0.05 | 0.04 | 0.05 | 0.0 | 0.17 | 0.08 | 0.0 |

| Impurity | Impurity 10 | | Unknown | Unknown | Unknown | | | |
|---|---|---|---|---|---|---|---|---|
| relative retention | 1.13 | | 1.26 | 1.35 | 1.63 | | | |
| time | | | | | | | | |
| amount% | 0.21 | 0.13 | 0.05 | 0.04 | | | | |

### Reference Example 1

### Preparation of impurities 6-10 in the crude compound of formula II

23 g of Sodium Micafungin was prepared from the crude compound of formula I as raw material obtained in Example 1 according to the preparation and purification process for Micafungin in WO2004014879.

About 5 g of Micafungin sodium was dissolved in 50 ml of pure water, divided into 10 batches, and prepared through preparative column (Prep Nova-Pak^{®}HR 7.8 * 300 mm). Acetonitrile: water = 70: 45 was used for elution. Elutes, the relative retention times of which are 0.71-0.74, 0.90-0.93, 1.08-1.10, and 1.11-1.13, were collected, concentrated, and dried, respectively. The impurities corresponding to the above relative retention times were determined as impurity 6, a mixture of impurities 7 and 8, impurity 9 and impurity 10, based on MS and ¹H-NMRanalysis. Their structures are shown in Formula IV, Formula V, Formula VI, Formula VII and Formula VIII, respectively.

### Preparation Conditions:

HPLC preparative column: Prep Nova-Pak^{®}HR 7.8 * 300 mm
Mobile phase: acetonitrile: water = 70: 45
Elution program: isocratic
Flow rate: 4 ml / min
Column temperature: 35°C
Running time: 40 min
Diluent: water

### Example 18

### Preparation of the high purity compound of formula II from the high purity compound of formula I and amount of each single impurity being less than 0.1%

The compound of formula II was synthesized from the compound of formula I according to the process for Micafungin synthesis in WO2004014879.

The compound of formula I obtained in Example 4 of the present application (HPLC purity of which was 99.57% (1.00 g, 1.07 mmol)) was dissolved in 12 ml of DMF. The resulting solution was cooled to below 0°C in an ice bath. Diisopropylethylamine (0.22 g, 1.67 mmol) was added, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into the reaction liquid at the end of the reaction, stirred for another 1 hour, and filtered, so as to give Micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity of Micafungin diisopropylethylamine was determined as 99.49% by HPLC, and the yield was 95.1 %. The amount of main relevant impurities is shown in Table 20.

**Table 20**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.92 | 0.96 | 1.09 |
| amount% | 0.01 | 0.02 | 0.02 | 0.05 | 0.0 | 0.09 | 0.06 | 0.0 |
| Impurity | Impurity 10 | | Unknown | Unknown | Unknown | | | |
| relative retention time | 1.13 | | 1.26 | 1.35 | 1.63 | | | |
| amount% | 0.09 | | 0.08 | 0.05 | 0.04 | | | |

### Example 19

### Preparation of the high purity compound of formula II from the high purity compound of formula I and amount of each single impurity being less than 0.1%

The compound of formula II was synthesized from the compound of formula I according to the process for Micafungin synthesis in WO2004014879.

The compound of formula I obtained in Example 5 of the present application (HPLC purity of which was 99.81 % (1.00 g, 1.07 mmol)) was dissolved in 12 ml of DMF. The resulting solution was cooled to below 0°C in an ice bath. Diisopropylethylamine (0.22 g, 1.67 mmol) was added, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into the reaction liquid at the end of the reaction, stirred for another 1 hour, and filtered, so as to give Micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity of Micafungin diisopropylethylamine was determined as 99.80% by HPLC, and the yield was 97.5%. The amount of main relevant impurities is shown in Table 21.

**Table 21**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.92 | 0.96 | 1.09 |
| amount% | 0.0 | 0.0 | 0.0 | 0.03 | 0.0 | 0.05 | 0.04 | 0.0 |
| Impurity | Impurity 10 | | Unknown | Unknown | Unknown | | | |
| relative retention time | 1.13 | | 1.26 | 1.35 | 1.63 | | | |
| amount% | 0.05 | | 0.02 | 0.01 | 0.0 | | | |

### Comparative Example 3

### Preparation of the compound of formula II from the crude compound of formula I

The compound of formula II was synthesized from the compound of formula I according to the process for Micafungin synthesis in WO2004014879.

The compound of formula I obtained in Example 1 of the present application (HPLC purity of which was 97.51% (10.7 mmol)) was dissolved in 12 ml of DMF. The resulting solution was cooled to below 0°C in an ice bath. Diisopropylethylamine (2.2 g, 16.7 mmol) was added, and the temperature was kept at 0°C. MKC-8 (1-[4-[5-(4-pentyloxyphenyl)isoxazol-3-yl]benzoyloxy]-1H-1,2,3-benzotriazole) (5.3 g, 11.4 mmol) was slowly added, and the reaction was warmed to 2-6°C, and maintained for 4 hours. 600 ml of ethyl acetate was added directly into the reaction liquid at the end of the reaction, stirred for another 1 hour, and filtered, so as to give Micafungin diisopropylethylamine. The salt was dissolved in 300 ml of acetone and 300 ml of ethyl acetate, starching and filtered. Micafungin diisopropylethylamine was dried in vacuo to remove residual organic solvent. The purity of Micafungin diisopropylethylamine was determined as 95.75% by HPLC, and the mole yield was 75.2%. Chromatographic peaks before 5 mins in HPLC Pattern (see Fig. 4) corresponded to solvent and reaction by-product, 1-hydroxybenzotriazole (HOBT), which can be readily removed by the purification process reported in WO2004014879. The amount of main relevant impurities is shown in Table 22.

**Table 22**

| Impurity | Unknown | Unknown | Unknown | Unknown | Impurity 6 | Impurity 7 and 8 | | Impurity 11 | Impurity 9 |
|---|---|---|---|---|---|---|---|---|---|
| relative retention time | 0.44 | 0.54 | 0.61 | 0.64 | 0.73 | 0.92 | | 0.96 | 1.09 |
| amount% | 0.21 | 0.18 | 0.04 | 0.03 | 0.07 | 0.29 | | 0.26 | 0.81 |
| Impurity | Impurity 10 | | Unknown | Unknown | Unknown | Unknown | Unknown | | |
| relative retention time | 1.13 | | 1.17 | 1.27 | 1.39 | 1.49 | 1.78 | | |
| amount% | 1.43 | | 0.17 | 0.28 | 0.38 | 0.06 | 0.04 | | |

### Example 20

### Preparation of high purity Micafungin sodium from high purity micafungin diisopropylethylamine

Micafungin sodium was prepared from Micafungin diisopropylethylamine according to the process in Example 6 in WO2004014879.

Micafungin diisopropylethylamine (0.97 mmol) prepared in Example 16 of the present application was dissolved in 15 ml of 75% aqueous methanol. The solution comprising Micafungin diisopropylethylamine was loaded onto 30 ml of UBK510L ion exchange resin. The loaded resin was eluted by using 75% of aqueous methanol until the concentration of Micafungin sodium was less than 1.0 g/L. The pH of collected liquid was adjusted to 6.0 using 0.1 M NaOH. The collected liquid was diluted by pure watertill the concentration of methanol was 35%. The resulting solution was loaded on 50 ml of pretreated HP20ss macropore adsorption resin for adsorption. The loaded resin was washed by using 100 ml of 35% aqueous methanol, and then eluted by using 200 ml of 80% aqueous methanol. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.90 mmol), and the yield was 93%. The portions comprising Micafungin sodium were pooled and distilled under reduced pressure in darkness to give a solid. The solid was dried in vacuo, and the purity of Micafungin sodium was determined as 99.00% by HPLC, wherein the reaction by-product, 1-hydroxybenzotriazole (HOBT) was completely removed. The amount of main relevant impurities is shown in Table 23.

**Table 23**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.91 | 0.95 | 1.09 |
| amount% | 0.0 | 0.0 | 0.02 | 0.02 | 0.19 | 0.20 | 0.08 | 0.13 |
| Impurity | Impurity 10 | | Unknown | Unknown | Unknown | | | |
| relative retention time | 1.12 | | 1.20 | 1.26 | 1.35 | | | |
| amount% | 0.23 | | 0.04 | 0.09 | 0.0 | | | |

### Comparative Example 4

### Preparation of Micafungin sodium from micafungin diisopropylethylamine prepared in Comparative Example 3

Micafungin sodium was prepared from Micafungin diisopropylethylamine according to the process in Example 6 in WO2004014879.

Micafungin diisopropylethylamine (0.97 mmol) prepared in Comparative Example 3 of the present application was dissolved in 15 ml of 75% aqueous methanol. The solution comprising Micafungin diisopropylethylamine was loaded onto 30 ml of UBK510L ion exchange resin. The loaded resin was eluted by using 75% of aqueous methanol until the concentration of Micafungin sodium was less than 1.0 g/L. The pH of collected liquid was adjusted to 6.0 using 0.1 mol/L NaOH. The collected liquid was diluted by pure water, so that the concentration of methanol was 35%. The resulting solution was loaded on 50 ml of pretreated HP20ss macropore adsorption resin for adsorption. The loaded resin was washed by using 100 ml of 35% aqueous methanol, and then eluted by usng 200 ml of 80% aqueous methanol. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.88 mmol), and the yield was 91%. The portions comprising Micafungin sodium were pooled and distilled under reduced pressure in darkness to give a solid. The solid was dried in vacuo, and the purity of Micafungin sodium was determined as 96.17% by HPLC, wherein the reaction by-product, 1-hydroxybenzotriazole (HOBT) was completely removed. The amount of main relevant impurities is shown in Table 24.

**Table 24**

| Impurity | Unknown | Unknown | Unknown | Unknown | Impurity 6 | Impurity 7 and 8 | | Impurity 11 | Impurity 9 |
|---|---|---|---|---|---|---|---|---|---|
| relative retention time | 0.4 | 0.54 | 0.62 | 0.64 | 0.72 | 0.91 | | 0.95 | 1.09 |
| amount% | 0.15 | 0.12 | 0.03 | 0.03 | 0.05 | 0.30 | | 0.26 | 0.81 |
| Impurity | Impurity 10 | | Unknown | Unknown | Unknown | Unknown | Unknown | | |
| relative retention time | 1.12 | | 1.19 | 1.26 | 1.38 | 1.49 | 1.78 | | |
| amount% | 1.43 | | 0.16 | 0.29 | 0.20 | 0.0 | 0.0 | | |

From Example 20 and Comparative Example 4, it is clear that, through simple purification procedure, Micafungin sodium (purity of 98.99%) can be obtained from high purity Micafungin diisopropylethylamine, which is prepared from the high purity compound of formula I (purity of 99.0%). However, if the compound of formula I, purity of which is 97.51 %, prepared according to Example 1 of US Pat No. 5376634 is used to prepare the high purity micafungin diisopropylethylamine, the purity of Micafungin sodium obtained from Micafungin diisopropylethylamine is merely 96.17%. Therefore, micafungin sodium prepared from the high purity compound of formula I is superior in purity.

### Example 21

### Preparation of high purity Micafungin sodium from high purity micafungin diisopropylethylamine

Micafungin sodium was prepared from Micafungin diisopropylethylamine according to the process in Example 6 in WO2004014879.

Micafungin diisopropylethylamine (0.98 mmol) prepared in Example 17 of the present application was dissolved in 15 ml water. The solution comprising Micafungin diisopropylethylamine was loaded onto 30 ml of UBK510L ion exchange resin. The loaded resin was eluted by using water until the concentration of Micafungin sodium was less than 1.0 g/L. The pH of collected liquid was adjusted to 6.0 using 0.1 mol/L NaOH. The collected liquid was loaded on 40 ml of pretreated SP-207ss macropore adsorption resin for adsorption. The loaded resin was washed by using 120 ml of 35% aqueous ethanol, and then eluted by using 150 ml of 80% aqueous ethanol. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.92 mmol), and the yield was 93.9%. The portions comprising Micafungin sodium were pooled and distilled under reduced pressure in darkness to give a solid. The solid was dried in vacuo, and the purity of Micafungin sodium was determined as 99.40% by HPLC, wherein the reaction by-product, 1-hydroxybenzotriazole (HOBT) was completely removed. The amount of main relevant impurities is shown in Table 25.

**Table 25**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.91 | 0.95 | 1.09 |
| amount% | 0.0 | 0.02 | 0.02 | 0.04 | 0.0 | 0.15 | 0.07 | 0.0 |
| Impurity | Impurity 10 | | Unknown | Unknown | Unknown | | | |
| relative retention time | 1.12 | | 1.20 | 1.26 | 1.35 | | | |
| amount% | 0.21 | | 0.02 | 0.07 | 0.0 | | | |

### Comparative Example 5

### Preparation of Micafungin sodium from micafungin diisopropylethylamine prepared in Comparative Example 3

Micafungin sodium was prepared from Micafungin diisopropylethylamine according to the process in Example 6 in WO2004014879.

Micafungin diisopropylethylamine (0.98 mmol) prepared in Comparative Example 3 of the present application was dissolved in 15 ml of water. The solution comprising Micafungin diisopropylethylamine was loaded onto 30 ml of UBK510L ion exchange resin. The loaded resin was eluted by using pure water until the concentration of Micafungin sodium was less than 1.0 g/L. The pH of collected liquid was adjusted to 6.0 using 0.1 mol/L NaOH. The collected liquid was loaded on 40 ml of pretreated SP-207ss macropore adsorption resin for adsorption. The loaded resin was washed by using 120 ml of 35% aqueous ethanol, and then eluted by usng 150 ml of 80% aqueous ethanol. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.84 mmol), and the yield was 86.1 %. The portions comprising Micafungin sodium were pooled and distilled under reduced pressure in darkness to give a solid. The solid was dried in vacuo, and the purity of Micafungin sodium was determined as 96.16% by HPLC, wherein the reaction by-product, 1-hydroxybenzotriazole (HOBT) was completely removed. The amount of main relevant impurities is shown in Table 26.

**Table 26**

| Impurity | Unknown | Unknown | Unknown | Unknown | Impurity 6 | Impurity 7 and 8 | | Impurity 11 | Impurity 9 |
|---|---|---|---|---|---|---|---|---|---|
| relative retention time | 0.4 | 0.54 | 0.62 | 0.64 | 0.72 | 0.91 | | 0.95 | 1.09 |
| amount% | 0.13 | 0.10 | 0.02 | 0.04 | 0.09 | 0.30 | | 0.26 | 0.81 |
| Impurity | Impurity 10 | | Unknown | Unknown | Unknown | Unknown | Unknown | | |
| relative retention time | 1.12 | | 1.19 | 1.26 | 1.38 | 1.49 | 1.78 | | |
| amount% | 1.43 | | 0.16 | 0.29 | 0.20 | 0.01 | 0.0 | | |

From Example 21 and Comparative Example 5, it is clear that, through simple purification procedure, Micafungin sodium (purity of 99.40%) can be obtained from high purity Micafungin diisopropylethylamine, which is prepared from the high purity compound of formula I (purity of 99.23%). However, if the compound of formula I, purity of which is 97.51 %, prepared according to Example 1 of US Pat No. 5376634 is used to prepare the high purity Micafungin diisopropylethylamine, the purity of micafungin sodium obtained from Micafungin diisopropylethylamine is merely 96.16% with the same purification process. Therefore, micafungin sodium prepared from the high purity compound of formula I is superior in purity.

### Example 22

### Preparation of high purity micafungin sodium and amount of each single impurity being less than 0.1% from high purity micafungin diisopropylethylamine

Micafungin sodium was prepared from Micafungin diisopropylethylamine according to the process in Example 6 in WO2004014879.

Micafungin diisopropylethylamine (1.01 mmol) prepared in Example 18 of the present application was dissolved in 15 ml water. The solution comprising Micafungin diisopropylethylamine was loaded onto 30 ml of UBK510L ion exchange resin. The loaded resin was eluted by using water until the concentration of Micafungin sodium was less than 1.0 g/L. The pH of collected liquid was adjusted to 6.0 using 0.1 mol/L NaOH. The collected liquid was loaded on 40 ml of pretreated SP-700 macropore adsorption resin for adsorption. The loaded resin was washed by using 100 ml of 30% aqueous acetone, and then eluted by usng 150 ml of 80% aqueous acetone. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.92 mmol), and the yield was 91.1%. The portions comprising Micafungin sodium were pooled and distilled under reduced pressure in darkness to give a solid. The solid was dried in vacuo, and the purity of Micafungin sodium was determined as 99.79% by HPLC, wherein the reaction by-product, 1-hydroxybenzotriazole (HOBT) was completely removed. The amount of main relevant impurities is shown in Table 27.

**Table 27**

| Impurity | Unknown | Unknown | Unknown | Unknown | Impurity 6 | Impurity 7 and 8 | Impurity 11 | Impurity 9 |
|---|---|---|---|---|---|---|---|---|
| relative retention time | 0.4 | 0.54 | 0.57 | 0.62 | 0.72 | 0.91 | 0.95 | 1.09 |
| amount% | 0.0 | 0.0 | 0.0 | 0.0 | 0.0 | 0.09 | 0.03 | 0.0 |
| Impurity | Impurity 10 | | Unknown | Unknown | Unknown | | | |
| relative retention time | 1.12 | | 1.20 | 1.26 | 1.35 | | | |
| amount% | 0.05 | | 0.04 | 0.0 | 0.0 | | | |

### Comparative Example 6

### Preparation of Micafungin sodium from diisopropylethylamine prepared in Comparative Example 3

Micafungin sodium was prepared from Micafungin diisopropylethylamine according to the process in Example 6 in WO2004014879.

Micafungin diisopropylethylamine (1.01 mmol) prepared in Comparative Example 3 of the present application was dissolved in 15 ml of water. The solution comprising Micafungin diisopropylethylamine was loaded onto 30 ml of UBK510L ion exchange resin. The loaded resin was eluted by using pure water until the concentration of Micafungin sodium was less than 1.0 g/L. The pH of collected liquid was adjusted to 6.0 using 0.1 mol/L NaOH. The collected liquid was loaded on 40 ml of pretreated SP-700 macropore adsorption resin for adsorption. The loaded resin was washed by using 100 ml of 30% aqueous acetone, and then eluted by usng 150 ml of 80% aqueous acetone. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.83 mmol), and the yield was 82.1 %. The portions comprising Micafungin sodium were pooled and distilled under reduced pressure in darkness to give a solid. The solid was dried in vacuo, and the purity of Micafungin sodium was determined as 96.29% by HPLC, wherein the reaction by-product, 1-hydroxybenzotriazole (HOBT) was completely removed. The amount of main relevant impurities is shown in Table 28.

**Table 28**

| Impurity | Unknown | Unknown | Unknown | Unknown | Impurity 6 | Impurity 7 and 8 | | Unknown | Impurity 9 |
|---|---|---|---|---|---|---|---|---|---|
| relative retention time | 0.4 | 0.54 | 0.62 | 0.64 | 0.72 | 0.91 | | 0.95 | 1.09 |
| amount% | 0.13 | 0.10 | 0.02 | 0.04 | 0.09 | 0.28 | | 0.25 | 0.80 |

| Impurity | Impurity 10 | | Unknown | Unknown | Unknown | Unknown | Unknown | | |
|---|---|---|---|---|---|---|---|---|---|
| relative retention time | 1.12 | | 1.19 | 1.26 | 1.38 | 1.49 | 1.78 | | |
| amount% | 1.40 | | 0.16 | 0.29 | 0.15 | 0.00 | 0.0 | | |

From Example 22 and Comparative Example 6, it is clear that, through simple purification procedure, Micafungin sodium (purity of 99.79%) can be obtained from high purity Micafungin diisopropylethylamine, which is prepared from the high purity compound of formula I (purity of 99.57%). However, if the compound of formula I, purity of which is 97.51 %, prepared according to Example 1 of US Pat No. 5376634 is used to prepare the high purity Micafungin diisopropylethylamine, the purity of Micafungin sodium obtained from Micafungin diisopropylethylamine is merely 96.29% with the same purification process. Therefore, micafungin sodium prepared from the high purity compound of formula I is superior in purity.

### Example 23

### Preparation of high purity micafungin sodium from high purity micafungin diisopropylethylamine and amount of each single impurity being less than 0.1%

Micafungin sodium was prepared from Micafungin diisopropylethylamine according to the process in Example 6 in WO2004014879.

Micafungin diisopropylethylamine (1.04 mmol) prepared in Example 19 of the present application was dissolved in 15 ml water. The solution comprising Micafungin diisopropylethylamine was loaded onto 25 ml of UBK510L ion exchange resin. The loaded resin was eluted by using water until the concentration of Micafungin sodium was less than 1.0 g/L. The pH of collected liquid was adjusted to 6.0 using 0.1 mol/L NaOH. The collected liquid was loaded on 40 ml of pretreated HP2MG macropore adsorption resin for adsorption. The loaded resin was washed by using 200 ml of 30% aqueous ethanol, and then eluted by usng 200 ml of 70% aqueous ethanol. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.92 mmol), and the yield was 88.5%. The portions comprising Micafungin sodium were pooled and distilled under reduced pressure in darkness to give a solid. The solid was dried in vacuo, and the purity of Micafungin sodium was determined as 99.88% by HPLC, wherein the reaction by-product, 1-hydroxybenzotriazole (HOBT) was completely removed. The amount of main relevant impurities is shown in Table 29.

**Table 29**

| Impurity | Unknown | Unknown | Unknown | Unknown | Impurity 6 | Impurity 7 and 8 | Unknown | Impurity 9 |
|---|---|---|---|---|---|---|---|---|
| relative retention time | 0.4 | 0.54 | 0.57 | 0.62 | 0.72 | 0.91 | 0.95 | 1.09 |
| amount% | 0.0 | 0.0 | 0.0 | 0.0 | 0.0 | 0.04 | 0.03 | 0.0 |

| Impurity | Impurity 10 | | Unknown | Unknown | Unknown | | | |
|---|---|---|---|---|---|---|---|---|
| relative retention time | 1.12 | | 1.20 | 1.26 | 1.35 | | | |
| amount% | 0.05 | | 0.0 | 0.0 | 0.0 | | | |

### Comparative Example 7

### Preparation of Micafungin sodium from diisopropylethylamine prepared in Comparative Example 3

Micafungin sodium was prepared from Micafungin diisopropylethylamine according to the process in Example 6 in WO2004014879.

Micafungin diisopropylethylamine (1.04 mmol) prepared in Comparative Example 3 of the present application was dissolved in 15 ml of water. The solution comprising Micafungin diisopropylethylamine was loaded onto 25 ml of UBK510L ion exchange resin. The loaded resin was eluted by using pure water until the concentration of Micafungin sodium was less than 1.0 g/L. The pH of collected liquid was adjusted to 6.0 using 0.1 mol/L NaOH. The collected liquid was loaded on 40 ml of pretreated HP2MG macropore adsorption resin for adsorption. The loaded resin was washed by using 200 ml of 30% aqueous ethanol, and then eluted by usng 200 ml of 70% aqueous ethanol. Elute was collected when the concentration of Micafungin sodium was more than 0.5 g/L, and the collection was stopped when the concentration of Micafungin sodium was less than 0.5 g/L. All of the portions comprising qualified concentration of Micafungin sodium were collected. The obtained Micafungin sodium was quantitatively analyzed by HPLC (0.86 mmol), and the yield was 82.7%. The portions comprising Micafungin sodium were pooled and distilled under reduced pressure in darkness to give a solid. The solid was dried in vacuo, and the purity of Micafungin sodium was determined as 96.29% by HPLC, wherein the reaction by-product, 1-hydroxybenzotriazole (HOBT) was completely removed. The amount of main relevant impurities is shown in Table 30.

**Table 30**

| Impurity | Unknown | Unknown | Unknown | Unknown | Impurity 6 | Impurity 7 and 8 | | Unknown | Impurity 9 |
|---|---|---|---|---|---|---|---|---|---|
| relative retention time | 0.4 | 0.54 | 0.62 | 0.64 | 0.72 | 0.91 | | 0.95 | 1.09 |
| amount% | 0.13 | 0.10 | 0.02 | 0.04 | 0.09 | 0.28 | | 0.25 | 0.80 |

| Impurity | Impurity 10 | | Unknown | Unknown | Unknown | Unknown | Unknown | | |
|---|---|---|---|---|---|---|---|---|---|
| relative retention time | 1.12 | | 1.19 | 1.26 | 1.38 | 1.49 | 1.78 | | |
| amount% | 1.40 | | 0.16 | 0.29 | 0.15 | 0.00 | 0.0 | | |

From Example 23 and Comparative Example 7, it is clear that, through simple purification procedure, Micafungin sodium (purity of 99.88%) can be obtained from high purity Micafungin diisopropylethylamine, which is prepared from the high purity compound of formula I (purity of 99.81%). However, if the compound of formula I, purity of which is 97.51 %, prepared according to Example 1 of US Pat No. 5376634 is used to prepare the high purity Micafungin diisopropylethylamine, the purity of Micafungin sodium obtained from Micafungin diisopropylethylamine is merely 96.29% with the same purification process. Therefore, micafungin sodium prepared from the high purity compound of formula I is superior in purity.

### Comparative Example 8

### Comparison with commercially available formulation in the impurity content

Commercially available formulations: Product Name: MYCAMINE
Generic name: Micafungin sodium for injection
Manufacturer: Astellas Toyama Corporation
Lot: 0901

**Table 31 Comparison of the high purity compound of formula II with commercially available formulation in the impurity content**

| Product | relative retention time | HPLC purity/HPLC content | |
|---|---|---|---|
| | | Commercially available formulation | Example 22 |
| Micafungin (compound of formula II) | 1.0 | 98.01% | 99.79 |
| Impurity 6 | 0.71-0.74 | 0.34% | - |
| Impurity 7 | 0.90-0.93 | 0.71% | 0.09 |
| Impurity 8 | 0.90-0.93 | | |
| Impurity 9 | 1.08-1.10 | 0.28 | - |
| Impurity 10 | 1.11-1.13 | 0.21 | 0.05 |
| Impurity 11 | 0.94-0.98 | 0.18 | 0.03 |

| | | | |
|---|---|---|---|
| -: means that the impurity could not be detected | | | |

From Table 31, it is clear that the purity of micafungin sodium obtained in the present invention is significantly higher than that of the commercially available formulation, wherein the amount of impurity 6, impurity 7, impurity 8, impurity 9, and impurity 10 is by far less than that in the commercially available formulation. See Figure 5 for HPLC pattern of micafungin sodium obtained in the present invention, and see Figure 6 for the commercially available formulation.

### Example 24

### Preparation of a pharmaceutical composition comprising compound I

| High purity compound I | Lactose | anhydrous citric acid | NaOH |
|---|---|---|---|
| 2.5 g | 20 g | q.s. | q.s. |

Lactose was dissolved into pure water (200 ml) at the temperature lower than 50°C. After cooling below 20°C, into the lactose solution was added high purity compound I obtained in Example 3. The resulting solution was gently stirred to avoid bubbles. 2% aqueous citric acid (0.95 ml)was added, and then into the solution was added 0.4% aqueous NaOH (approximately 24 ml) for adjusting pH to 5.5. And then the resulting solution was diluted with pure water to produce a given volume (250 ml). The resulting solution was filled into 100 vials (the volume of which is 10 ml) with 2.5 ml for each. The solution in each vial was lyophilized using the lyophilizer according to conventional methods, so as to obtain lyophilized compositions, with each containing 25 mg of compound I.

The above examples are merely the preferred examples for the present invention, and such examples cannot be used to limit the scope of the invention. The substantial technical contents according to the present invention are broadly defined in the claims. And any entities or methods accomplished by others should be considered as the equivalents and fall within the scope as defined by the claims, if said entities or methods are the same as those defined by the claims.

## Claims

1. A high purity cyclopeptide compound of formula I, wherein the purity thereof is not less than 99.0%; wherein, R represents H or a cation capable of forming a pharmaceutically acceptable salt;

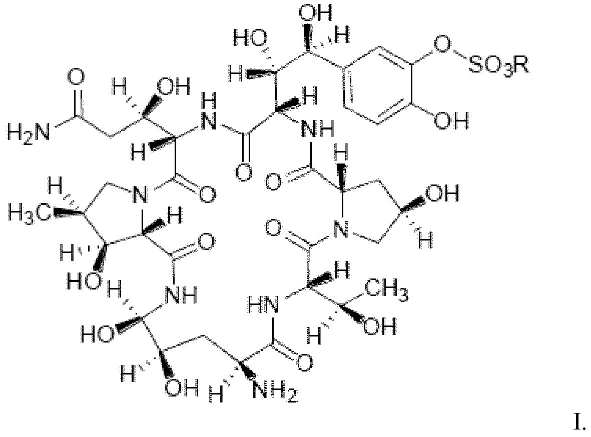

2. The high purity cyclopeptide compound according to claim 1, wherein the purity of the compound of formula I is determined by HPLC method.

3. The high purity cyclopeptide compound according to claim 2, wherein the HPLC method is listed as follows:
Chromatography column: ACE 3 AQ, 150 × 4.6mm, 3µm;
Mobile phase: A: 1000 ml of water, 10 ml of methanol, 100 µl of trifluoroacetic acid;
B: 600 ml of water, 400ml of methanol, 100µl of trifluoroacetic acid;
Flow rate: 0.55 ml/min;
Column temperature: 50°C;
Gradient:
| time | Mobile phase A | Mobile phase B |
|---|---|---|
| min | % | % |
| 0 | 100 | 0 |
| 25 | 100 | 0 |
| 55 | 55 | 45 |
| 56 | 0 | 100 |
| 61 | 0 | 100 |
| 62 | 100 | 0 |
| 70 | 100 | 0 |
Diluent: pure water;
Injector temperature: 5°C;
Detection wavelength: 225 nm;

4. The high purity cyclopeptide compound according to claim 1, wherein the amount of impurity A in the high purity cyclopeptide compound is not more than 0.25%, and the relative retention time of impurity A in HPLC is around 0.45.

5. The high purity cyclopeptide compound according to claim 1, wherein the amount of impurity B in the high purity cyclopeptide compound is not more than 0.25%, and the relative retention time of impurity B in HPLC is around 0.65.

6. The high purity cyclopeptide compound according to claim 1, wherein the amount of impurity C in the high purity cyclopeptide compound is not more than 0.25%, and the relative retention time of impurity C in HPLC is around 0.88.

7. The high purity cyclopeptide compound according to claim 1, wherein the amount of impurity D in the high purity cyclopeptide compound is not more than 0.20%, and the relative retention time of impurity D in HPLC is around 1.08.

8. The high purity cyclopeptide compound according to claim 1, wherein the amount of impurity E in the high purity cyclopeptide compound is not more than 0.15%, and the relative retention time of impurity E in HPLC is around 1.29.

9. The high purity cyclopeptide compound according to claim 1, wherein the amount of impurity F in the high purity cyclopeptide compound is not more than 0.15%, and the relative retention time of impurity F in HPLC is around 1.92.

10. The high purity cyclopeptide compound according to claim 1, wherein the amount of any other relevant impurities in the high purity cyclopeptide compound is not more than 0.10%, and said other relevant impurities refer to impurities other than impurities A-F which may be present.

11. The high purity cyclopeptide compound according to any one of claims 1-10, wherein the purity of the compound of formula I and / or the amount of impurities is calculated as follows: the area under curve of the peak for the compound of formula I and / or a impurity in HPLC pattern is divided by the total area under curve of HPLC pattern.

12. A preparation method for the high purity cyclopeptide compound according to any one of claims 1-11, including the following steps:
(a) dissolving the crude compound of formula I into water or aqueous organic solvent (i), and controlling pH of the solution;
(b) obtaining the high purity cyclopeptide compound by reducing the temperature and / or adding organic solvent (i).

13. The preparation method according to claim 12, wherein, in step (a), pH of the solution is controlled at 2.0-5.0; preferably 3.5-4.5.

14. The preparation method according to claim 12, wherein, the concentration of compound of formula I in the solution of step (a) is 10 to 500 mg/ml.

15. The preparation method according to claim 12, wherein, in step (a) and / or (b), said organic solvent (i) is selected from C1-C4 lower alcohol; preferably selected from: methanol, ethanol, n-propanol, isopropanol, or a mixture thereof.

16. The preparation method according to claim 12, wherein step (a) - (b) can be repeated for one or more times.

17. Use of the high purity compound of formula I according to any one of claims 1-11, for preparing high purity compound of formula II

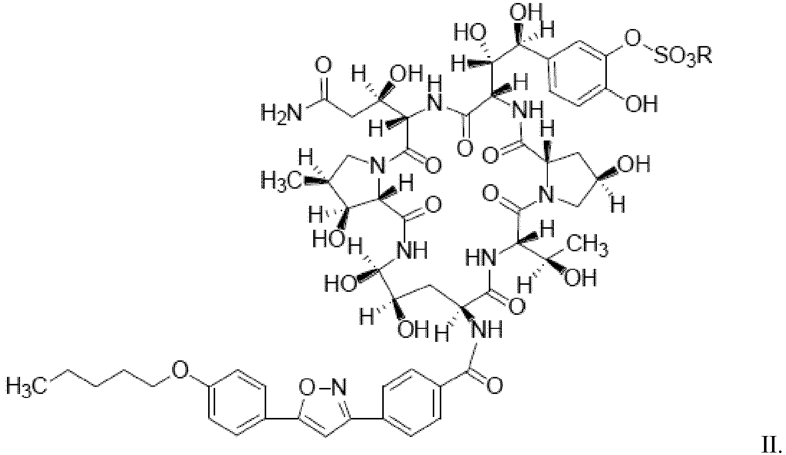

18. Use of the high purity compound of formula I according to any one of claims 1-11, for preparing medicaments for treating fungal infections.

19. A pharmaceutical composition comprising the high purity compound of formula I according to any one of claims 1-11 and a pharmaceutically acceptable carrier.

20. A preparation method for the pharmaceutical composition according to claim 19, including the following step:
the high purity compound of formula I according to any one of claims 1-11 is mixed with a pharmaceutically acceptable carrier, so as to obtain the pharmaceutical composition according to claim 19.

21. A high purity cyclopeptide compound of formula II, wherein the purity thereof is not less than 98.80%; wherein, R represents H or a cation capable of forming a pharmaceutically acceptable salt.

22. The compound of formula II according to claim 21, wherein the purity of the compound of formula II is determined by HPLC method.

23. The compound of formula II according to claim 21, wherein the amount of impurity 6, the structure of which is shown in Formula IV, in the compound of formula II is less than 0.3%; wherein, R represents H or a cation capable of forming a pharmaceutically acceptable salt;

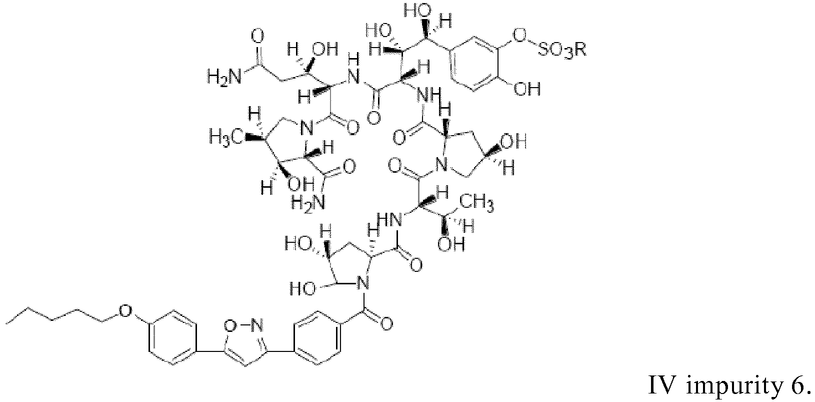

24. The compound of formula II according to claim 21, wherein the total amount of impurity 7, the structure of which is shown in Formula V, and impurity 8, the structure of which is shown in Formula VI, in the compound of formula II is less than 0.5%; wherein, R represents H or a cation capable of forming a pharmaceutically acceptable salt;

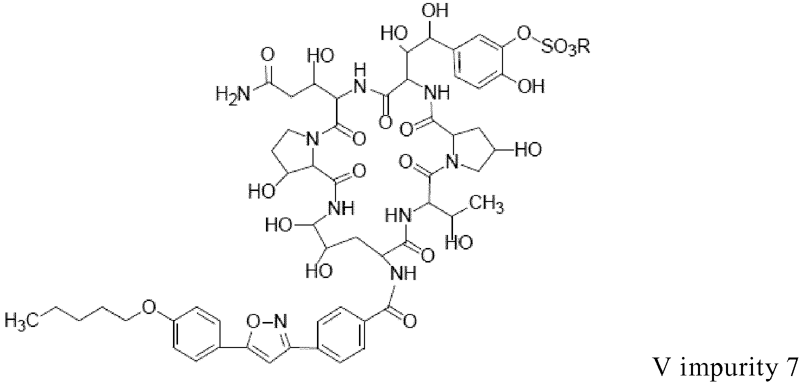

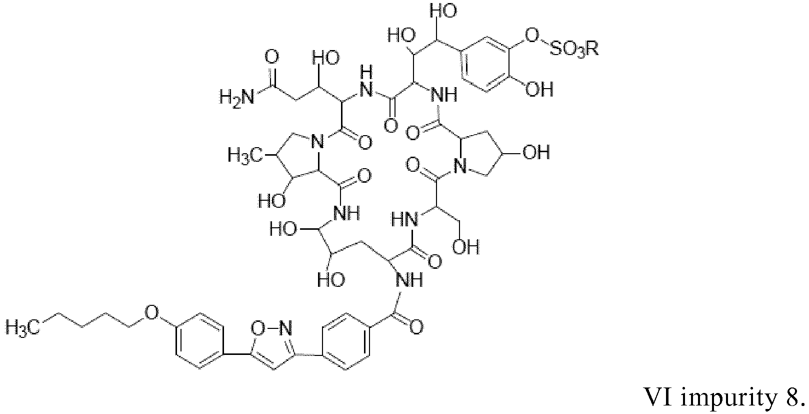

25. The compound of formula II according to claim 21, wherein the amount of impurity 9, the structure of which is shown in Formula VII, in the compound of formula II is less than 0.2%; wherein, R represents H or a cation capable of forming a pharmaceutically acceptable salt;

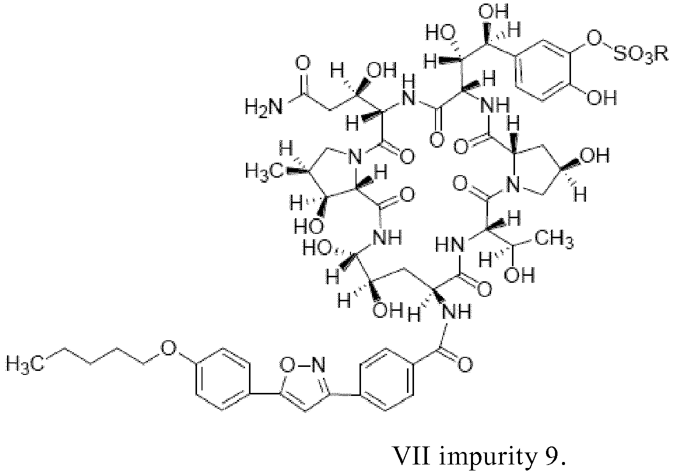

26. The compound of formula II according to claim 21, wherein the amount of impurity 10, the structure of which is shown in Formula VIII, in the compound of formula II is less than 0.2%; wherein, R represents H or a cation capable of forming a pharmaceutically acceptable salt;

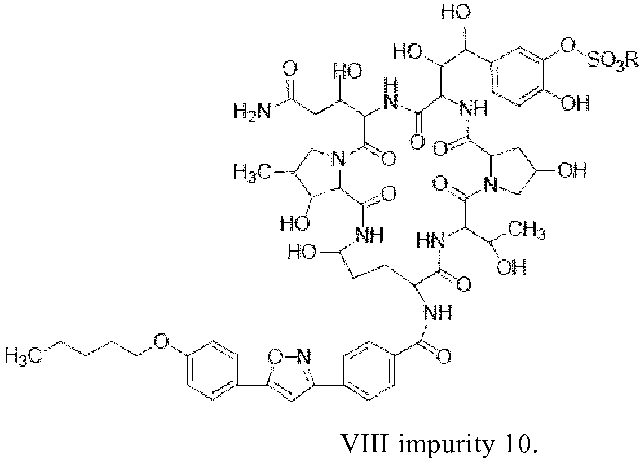

27. The compound of formula II according to claim 21, wherein the amount of impurity 11 in the compound of formula II is not more than 0.15%; and the relative retention time of impurity 11 in HPLC is around 0.96.

28. The compound of formula II according to claim 21, wherein the amount of any of other relevant impurities in the compound of formula II is not more than 0.02%; and said other relevant impurities refer to impurities other than impurities 6-11 which may be present.

29. A preparation method for the high-purity compound of formula II according to any one of claims 21-28, wherein the high-purity compound of formula I according to any one of claims 1-11 is used as raw material to prepare the compound of formula II.

30. Use of the high-purity compound of formula II according to any one of claims 21-28, for preparing medicaments for treating fungal infections.

31. A pharmaceutical composition comprising the high-purity compound of formula II according to any one of claims 21-28 and a pharmaceutically acceptable carrier.

32. A preparation method for the pharmaceutical composition according to claim 31, including the following step:
the high-purity compound of formula II according to any one of claims 21-28 is mixed with a pharmaceutically acceptable carrier, so as to obtain the pharmaceutical composition according to claim 31.
